# EUROPÄISCHE PATENTANMELDUNG

(11) **EP 1 969 933 A1**
(43) Veröffentlichungstag der Anmeldung: **17.09.2008**
(21) Anmeldenummer: 07005004.2
(22) Anmeldetag: 12.03.2007
(51) Int. Cl.: A01N 37/52, C07C 205/38, C07C 217/90, C07C 257/12

(54) **Dihalogenphenoxyphenylamidine und deren Verwendung als Fungizide**

(71) Anmelder: Bayer CropScience AG, 40789 Monheim (DE)
(72) Erfinder: Die Erfindernennung liegt noch nicht vor

(57) **Zusammenfassung**

Die vorliegende Erfindung betrifft Dihalogenphenoxyphenylamidine der allgemeinen Formel (I), ein Verfahren zu deren Herstellung, die Verwendung der erfindungsgemäßen Amidine zum Bekämpfen von unerwünschten Mikroorganismen, sowie ein Mittel zu diesem Zweck, umfassend die erfindungsgemäßen Phenoxyamidine. Weiterhin betrifft die Erfindung ein Verfahren zum Bekämpfen unerwünschter Mikroorganismen durch Ausbringen der erfindungsgemäßen Verbindungen auf die Mikroorganismen und/oder in deren Lebensraum.

## Beschreibung

Die vorliegende Erfindung betrifft Dihalogenphenoxyphenylamidine der allgemeinen Formel (I), ein Verfahren zu deren Herstellung, die Verwendung der erfindungsgemäßen Amidine zum Bekämpfen von unerwünschten Mikroorganismen, sowie ein Mittel zu diesem Zweck, umfassend die erfindungsgemäßen Phenoxyamidine. Weiterhin betrifft die Erfindung ein Verfahren zum Bekämpfen unerwünschter Mikroorganismen durch Ausbringen der erfindungsgemäßen Verbindungen auf die Mikroorganismen und/oder in deren Lebensraum.

WO-A-00/046 184 offenbart die Verwendung von Arylamidinen, unter anderem von N-Methyl-N-ethyl-N'-[4-(3,5-dichlorphenoxy)-2,5-xylyl]-formamidin als Fungizide.

WO-A-03/093 224 offenbart die Verwendung von Arylamidin-Derivaten als Fungizide.

WO-A-03/024 219 offenbart Fungizidzusammensetzungen umfassend wenigstens ein N2-Phenylamidin-Derivat in Kombination mit einem weiteren ausgewählten bekannten Wirkstoff.

WO-A-04/037239 offenbart Antimykotika auf der Basis von N2-Phenylamidin-Derivaten.

WO-A-05/089 547 offenbart Fungizidmischungen, umfassend wenigstens ein Arylamidin-Derivat in Kombination mit einem weiteren bekannten fungiziden Wirkstoff.

WO-A-05/120 234 offenbart Fungizidmischungen, umfassend wenigstens ein Phenylamidin-Derivat und ein weiteres ausgewähltes bekanntes Fungizid.

Die Wirksamkeit der im Stand der Technik beschriebenen Amidine ist gut, lässt jedoch in manchen Fallen zu wünschen übrig.

Der vorliegenden Erfindung liegt daher die Aufgabe zugrunde, Amidine mit einer verbesserten fungiziden Wirksamkeit zur Verfügung zu stellen.

Die Aufgabe wurde überraschenderweise gelöst durch Dihalogenphenoxyphenylamidine der Formel (I) 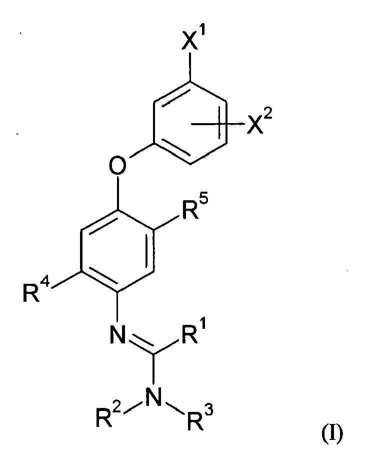 in welcher
- R¹: ausgewählt ist aus Wasserstoff; linearen, verzweigten C₁₋₁₂-Alkyl-, C₂₋₁₂-Alkenyl-, C₂₋₁₂-Alkinyl-Gruppen oder cyclischen C₃₋₁₂-Alkyl-, C₄₋₁₂-Alkenyl-, C₄₋₁₂-Alkinyl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und-CONR₂', substituiert sein können, wobei R' Wasserstoff oder eine C₁₋₁₂-Alkyl-Gruppe ist;
-SH; -SR", wobei R" eine lineare, verzweigte C₁₋₁₂-Alkyl-Gruppe oder cyclische C₁₋₁₂-Alkyl-Gruppe sein kann, die mit einer oder mehreren Gruppen, die ausgewählt sind aus -R', -X, -OR', -SR', -NR'₂, -SiR'₃, -COOR', -CN und-CONR₂' substituiert sein kann, worin R' die obigen Bedeutungen hat;
- R²: ausgewählt ist aus linearen, verzweigten C₁₋₁₂-Alkyl-, C₂₋₁₂-Alkenyl-, C₂₋₁₂-Alkinyl-, cyclischen C₃₋₁₂-Alkyl-, C₄₋₁₂-Alkenyl-, C₄₋₁₂-Alkinyl-Gruppen oder C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und-CONR₂' substituiert sein können, worin R' die obigen Bedeutungen hat;
- R³: ausgewählt ist aus -CN, -SH, -SR", -OR", -(C=O)-R", wobei R" die obigen Bedeutungen hat;
linearen, verzweigten C₂₋₁₂-Alkyl-, C₂₋₁₂-Alkenyl-, C₂₋₁₂-Alkinyl-, cyclischen C₃₋₁₂-Alkyl-, C₄₋₁₂-Alkenyl-, C₄₋₁₂-Alkinyl-Gruppen oder C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und-CONR₂' substituiert sein können, worin R' die obigen Bedeutungen hat;
oder in der
R² und R³,
- R² und R¹: oder
- R¹ und R³: mit den Atomen, an die sie gebunden sind, oder ggf. mit weiteren Atomen, ausgewählt aus N, O, P und S, einen vier- bis siebengliedrigen Ring bilden können, der wiederum mit R'-, OR'-, SR'-, NR'₂-, SiR'₃-Gruppen substituiert sein kann, worin R' die obigen Bedeutungen hat;
- R⁴ und R⁵: unabhängig voneinander ausgewählt sind aus der Gruppe bestehend aus Wasserstoff, -X, -CN, -SH, -SR", -OR", -(C=O)-R", wobei R" die obigen Bedeutungen hat;
linearen, verzweigten C₁₋₁₂-Alkyl-, C₂₋₁₂-Alkenyl-, C₂₋₁₂-Alkinyl-, cyclischen C₃₋₁₂-Alkyl-, C₄₋₁₂-Alkenyl-, C₄₋₁₂-Alkinyl-Gruppen oder C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und-CONR₂' substituiert sein können, worin R' die obigen Bedeutungen hat;
- X¹: ausgewählt ist aus der Gruppe bestehend aus F, Cl, Br und I;
- X²: sich entweder in 4- oder 5- Position der Phenyl-Gruppe befindet und unabhängig von X¹ ausgewählt ist aus der Gruppe bestehend aus F, Cl, Br und I;
wobei im Falle einer 3,5-Dihalosubstitution X¹ und X² nicht gleichzeitig Cl sind,
und deren Salze.

Ein weiterer Gegenstand der vorliegenden Erfindung betrifft ein Verfahren zum Herstellen der erfindungsgemäßen Dihalogenphenoxyphenylamidine umfassend wenigstens einen der folgenden Schritte (a) bis (j):
(a) Umsetzung von Nitrobenzolderivaten der Formel (III) mit Dihalogenphenolen der Formel (II) gemäß dem nachfolgenden Reaktionsschema: 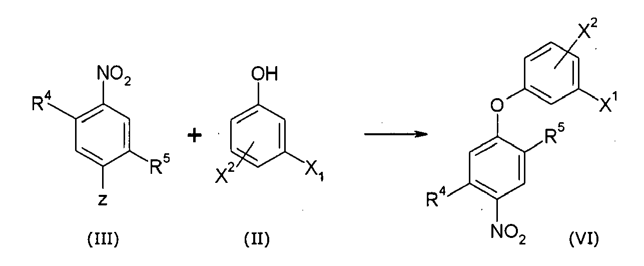
(b) Umsetzung von Nitrophenolderivaten der Formel (V) mit Dihalogenphenylderivaten der Formel (IV) gemäß dem nachfolgenden Reaktionsschema: 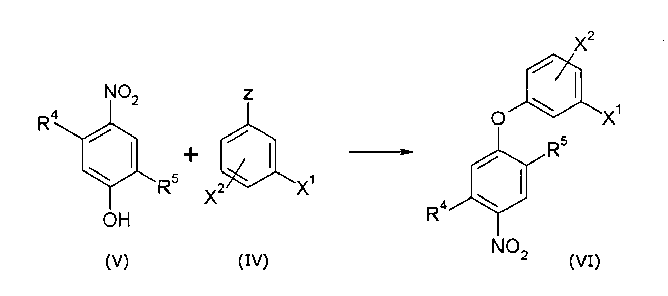
(c) Umsetzung von Anilinen der Formel (VII) mit Dihalogenphenolen der Formel (II) gemäß dem nachfolgenden Reaktionsschema: 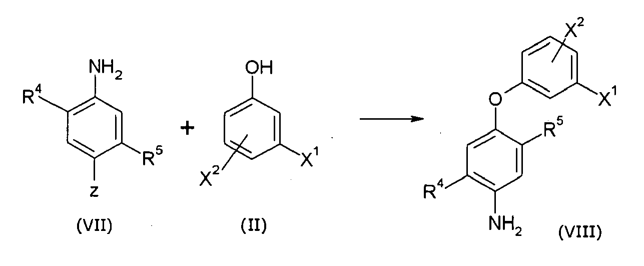
(d) Umsetzung von Aminophenolen der Formel (XII) mit Dihalogenphenylderivaten der Formel (IV) gemäß dem nachfolgenden Reaktionsschema: 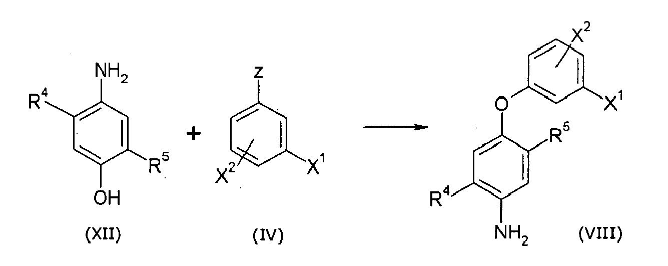
(e) Reduktion der Nitrophenylether der Formel (VI) zu Anilinethern der Formel (VIII) gemäß dem nachfolgenden Reaktionsschema: 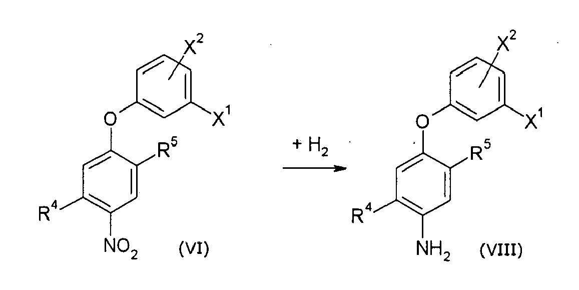
(f) Umsetzung der Anilinether der Formel (VIII) mit
   (i) Aminoacetalen der Formel (XIII) oder
   (ii) mit Amiden der Formel (XIV) oder
   (iii) mit Aminen der Formel (XV) in Gegenwart von Orthoestern der Formel (XVI)
   gemäß dem nachfolgenden Reaktionsschema: 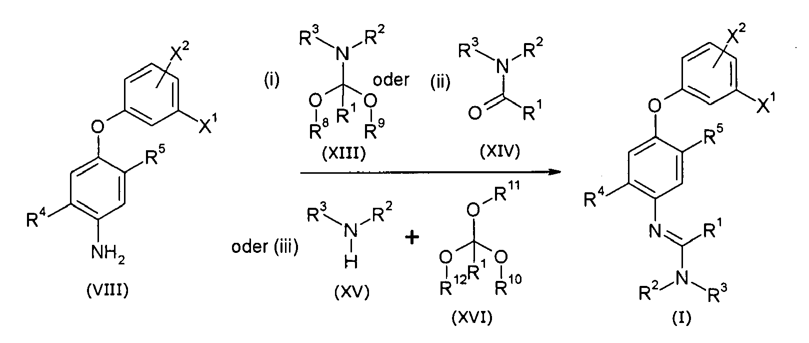
(g) Umsetzung der Aminophenole der Formel (XII) mit
   (i) Aminoacetalen der Formel (XIII) oder
   (ii) mit Amiden der Formel (XIV) oder
   (iii) mit Aminen der Formel (XV) in Gegenwart von Orthoestern der Formel (XVI)
   gemäß dem nachfolgenden Reaktionsschema: 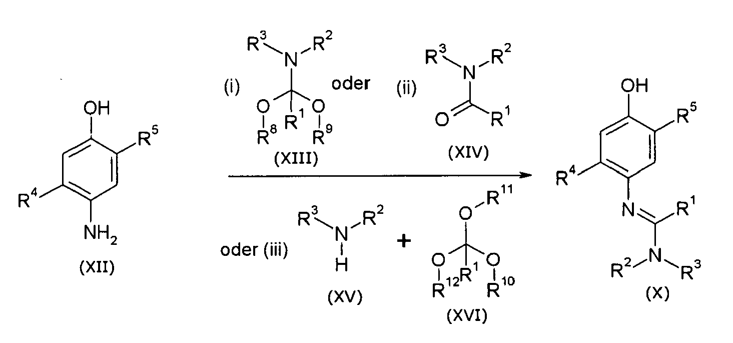
(h) Umsetzung der Aminophenole der Formel (VII) mit
   (i) Aminoacetalen der Formel (XIII) oder
   (ii) mit Amiden der Formel (XIV) oder
   (iii) mit Aminen der Formel (XV) in Gegenwart von Orthoestern der Formel (XVI)
   gemäß dem nachfolgenden Reaktionsschema: 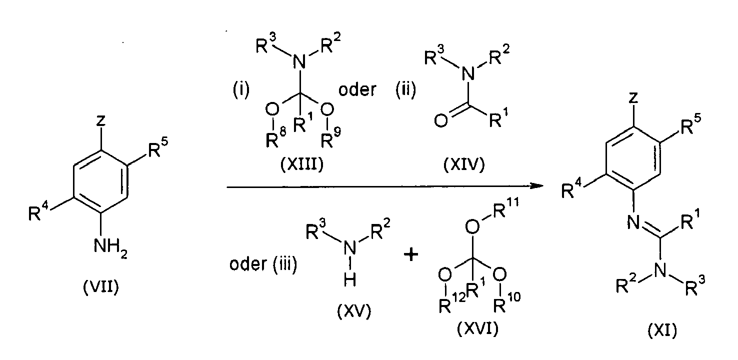
(i) Umsetzung von Amidinen der Formel (XI) mit dihalogensubstituierten Phenolen der Formel (II) gemäß dem nachfolgenden Reaktionsschema: 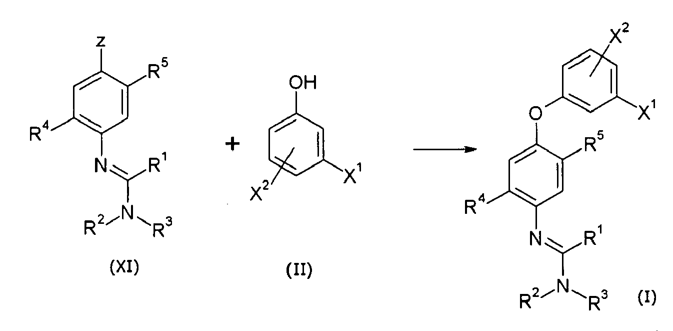
(j) Umsetzung von Amidinen der Formel (XI) mit dihalogensubstituierten Phenylderivaten der Formel (IV) gemäß dem nachfolgenden Reaktionsschema: 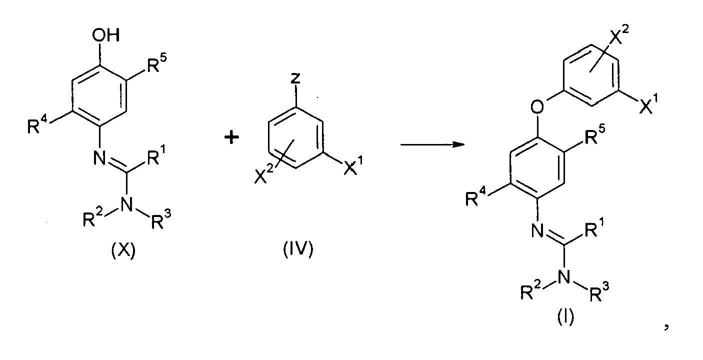 wobei in den obigen Schemata
   - Z: eine Austrittsgruppe ist;
   - X¹, X² und R' bis R⁵: die obigen Bedeutungen haben;
   - R⁶ bis R⁹: unabhängig voneinander ausgewählt sind aus der Gruppe bestehend aus Wasserstoff, C₁₋₁₂-Alkyl-, C₂₋₁₂-Alkenyl- C₂₋₁₂-Alkinyl- oder C₅₋₁₈-Aryl- oder C₇₋₁₉-Arylalkyl-Gruppen und gemeinsam mit den O-Atomen, an die sie gebunden sind einen fünf-, sechs- oder siebengliedrigen Ring bilden können;
   - R¹⁰, R¹¹ und R¹²: unabhängig voneinander ausgewählt sind aus der Gruppe bestehend aus Wasserstoff, C₁₋₁₂-Alkyl-, C₂₋₁₂-Alkenyl-, C₂₋₁₂-Alkinyl- oder C₅₋₁₈-Aryl- oder C₇₋₉-Arylalkyl-, C₇₋₁₉-Alkylaryl-Gruppen und jeweils R¹⁰ mit R¹², R¹⁰ mit R¹¹ oder R¹¹ mit R¹² gemeinsam mit den O-Atomen, an die sie gebunden sind einen fünf-, sechs- oder siebengliedrigen Ring bilden können.

Ein dritter Gegenstand der Erfindung sind Nitrophenylether der Formel (VI); 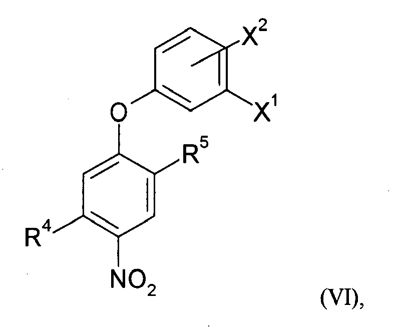 in der
R⁴, R⁵
- X¹ und X²: die obigen Bedeutungen haben; und wobei im Falle einer 3,5-Dihalosubstitution X¹ und X² nicht gleichzeitig Cl sind.

Ein vierter Gegenstand der Erfindung sind Anilinether der Formel (VIII) 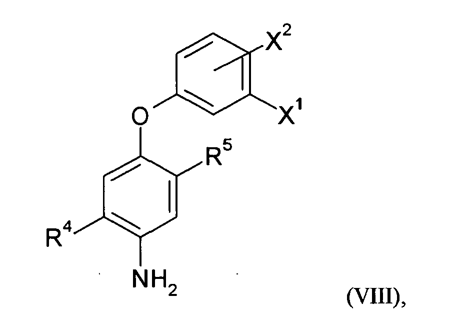 in der
R⁴, R⁵
- X¹ und X²: die obigen Bedeutungen haben; und wobei im Falle einer 3,5-Dihalosubstitution X¹ und X² nicht gleichzeitig C1 sind.

Ein fünfter Gegenstand der Erfindung sind Aminoacetale der Formel (XIII) 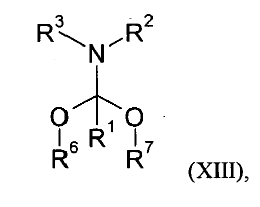 in der
- R¹ bis R⁷: die obigen Bedeutungen haben.

Ein sechster Gegenstand der Erfindung betrifft die medizinische oder nichtmedizinische Verwendung der erfindungsgemäßen Dihalogenphenoxyphenylamidine oder Mischungen dieser zum Bekämpfen unerwünschter Mikroorganismen.

Ein weiterer Gegenstand der Erfindung betrifft ein Mittel zum Bekämpfen unerwünschter Mikroorganismen, umfassend wenigstens ein Dihalogenphenoxyphenylamidin gemäß der vorliegenden Erfindung.

Außerdem betrifft die Erfindung ein Verfahren zum Bekämpfen unerwünschter Mikroorganismen, dadurch gekennzeichnet, dass die erfindungsgemäßen Dihalogenphenoxyphenylamidine auf die Mikroorganismen und/oder in deren Lebensraum ausgebracht werden.

Weiterhin betrifft die Erfindung ein Saatgut, welches mit wenigstens einem erfindungsgemäßen Dihalogenphenoxyphenylamidin behandelt wurde.

Ein letzter Gegenstand der Erfindung betrifft ein Verfahren zum Schutz von Saatgut vor unerwünschten Mikroorganismen durch Verwendung eines mit wenigstens einem Phenoxyamidin gemäß der vorliegenden Erfindung behandelten Saatgutes.

### Allgemeine Definitionen

Im Zusammenhang mit der vorliegenden Erfindung umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Im Zusammenhang mit der vorliegenden Erfindung bezeichnet die Gruppe --X ein Halogenatom, das ausgewählt ist aus Fluor, Chlor, Brom und Iod, vorzugsweise Fluor, Chlor und Brom, besonders bevorzugt aus Fluor und Chlor.

Mit einem oder mehreren Halogenatomen (-X) substituierte Alkyl-Gruppen sind beispielsweise ausgewählt aus Trifluormethyl (CF₃), Difluormethyl (CHF₂), CF₃CH₂, C1CH₂, CF₃CCl₂.

Alkyl-Gruppen sind im Zusammenhang mit der vorliegenden Erfindung, soweit nicht abweichend definiert, lineare, verzweigte oder ringförmige Kohlenwasserstoff-Gruppen, die optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₁-C₁₂-Alkyl umfasst den größten hierin definierten Bereich für einen Alkyl-Gruppe. Im Einzelnen umfasst diese Definition beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Alkenyl-Gruppen sind im Zusammenhang mit der vorliegenden Erfindung, soweit nicht abweichend definiert, lineare, verzweigte oder ringförmige Kohlenwasserstoff Gruppen, die wenigstens eine einfache Unsättigung (Doppelbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkenyl-Gruppen optional durch weitere Gruppen substituiert sein, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₂-C₁₂-Alkenyl umfasst den größten hierin definierten Bereich für einen AlkenylGruppe. Im Einzelnen umfasst diese Definition beispielsweise die Bedeutungen Vinyl; Allyl (2-Propenyl), Isopropenyl (1-Methylethenyl); But-1-enyl (Crotyl), But-2-enyl, But-3-enyl; Hex-1-enyl, Hex-2-enyl, Hex-3-enyl, Hex-4-enyl, Hex-5-enyl; Hept-1-enyl, Hept-2-enyl, Hept-3-enyl, Hept-4-enyl, Hept-5-enyl, Hept-6-enyl; Oct-1-enyl, Oct-2-enyl, Oct-3-enyl, Oct-4-enyl, Oct-5-enyl, Oct-6-enyl, Oct-7-enyl; Non-1-enyl, Non-2-enyl, Non-3-enyl, Non-4-enyl, Non-5-enyl, Non-6-enyl, Non-7-enyl, Non-8-enyl; Dec-1-enyl, Dec-2-enyl, Dec-3-enyl, Dec-4-enyl, Dec-5-enyl, Dec-6-enyl, Dec-7-enyl, Dec-8-enyl, Dec-9-enyl; Undec-1-enyl, Undec-2-enyl, Undec-3-enyl, Undec-4-enyl, Undec-5-enyl, Undec-6-enyl, Undec-7-enyl, Undec-8-enyl, Undec-9-enyl, Undec-10-enyl; Dodec-1-enyl, Dodec-2-enyl, Dodec-3-enyl, Dodec-4-enyl, Dodec-5-enyl, Dodec-6-enyl, Dodec-7-enyl, Dodec-8-enyl, Dodec-9-enyl, Dodec-10-enyl, Dodec-11-enyl; Buta-1,3-dienyl, Penta-1,3-dienyl.

Alkinyl-Gruppen sind im Zusammenhang mit der vorliegenden Erfindung, soweit nicht abweichend definiert, lineare, verzweigte oder ringförmige Kohlenwasserstoff Gruppen, die wenigstens eine zweifache Unsättigung (Dreifachbindung) enthalten und optional eine, zwei oder mehrere einfache oder zweifache Unsättigungen oder ein, zwei oder mehrere Heteroatome, die ausgewählt sind aus O, N, P und S aufweisen können. Außerdem können die erfindungsgemäßen Alkinyl-Gruppen optional durch weitere Gruppen substituiert sein, die ausgewählt sind aus -R', Halogen- (-X), Alkoxy-(-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine lineare, verzweigte oder cyclische C₁₋₁₂-Alkyl-Gruppe ist, die ein oder mehrere Heteroatome, ausgewählt aus N, O, P und S, aufweisen kann.

Die Definition C₂-C₁₂-Alkinyl umfasst den größten hierin definierten Bereich für einen Alkinyl-Gruppe. Im Einzelnen umfasst diese Definition beispielsweise die Bedeutungen Ethinyl (Acetylenyl); Prop-1-inyl und Prop-2-inyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S aufweisen können und optional durch weitere Gruppen substituiert sein können, die ausgewählt sind aus -R', Halogen- (-X), Alkoxy- (-OR'), Thioether-oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₅₋₁₈-Aryl umfasst den größten hierin definierten Bereich für einen Aryl-Gruppe mit 5 bis 18 Atomen. Im Einzelnen umfasst diese Definition beispielsweise die Bedeutungen Cyclopentadienyl, Phenyl, Cycloheptatrienyl, Cyclooctatetraenyl, Naphthyl und Anthracenyl.

Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Aralkyl-Gruppe umfasst den größten hierin definierten Bereich für einen Arylalkyl-Gruppe mit insgesamt 7 bis 19 Atomen in Gerüst und Alkylenkette. Im Einzelnen umfasst diese Definition beispielsweise die Bedeutungen Benzyl- und Phenylethyl-.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst oder der Alkylenkette durch eines oder mehrere Heteroatome, die ausgewählt sind aus O, N, P und S und optional durch weitere Gruppen substituiert sein können, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN), Acyl- (-(C=O)R') und Amid-Gruppen (-CONR₂'), wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann.

Die Definition C₇₋₁₉-Alkylaryl-Gruppe umfasst den größten hierin definierten Bereich für einen Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

Die Alkyl-, Alkenyl-, Alkinyl-, Aryl,- Alkaryl- und Aralkylgruppen können zudem ein oder mehrere Heteroatome aufweisen, die - soweit nicht abweichend definiert - ausgewählt sind aus N, O, P und S. Die Heteroatome ersetzen dabei die bezifferten Kohlenstoffatome.

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen offenbart und beansprucht.

Die erfindungsgemäßen Amidine sind Verbindungen der Formel (I) 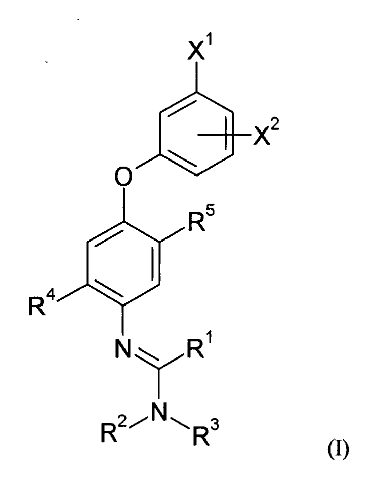 oder deren Salze, N-Oxide, Metallkomplexe und deren Stereoisomere.

In Formel (I) haben die Reste die im Folgenden definierten Bedeutungen. Die getroffenen Definitionen gelten für alle Zwischenprodukte gleichermaßen:
- R¹: ist ausgewählt aus:
- Wasserstoff;
- linearen, verzweigten C₁₋₁₂-Alkyl-, C₂₋₁₂-Alkenyl-, C₂₋₁₂-Alkinyl-Gruppen oder cyclischen C₃₋₁₂-Alkyl-, C₄₋₁₂-Alkenyl-, C₄₋₁₂-Alkinyl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN) und Amid-Gruppen (-CONR₂') substituiert sein können, wobei R' Wasserstoff oder eine C₁₋₁₂-Alkyl-Gruppe, vorzugsweise C₂₋₁₀-Alkyl-Gruppe, besonders bevorzugt C₃₋₈-Alkyl-Gruppe ist, die ein oder mehrere Heteroatome, ausgewählt aus N, O, P und S, aufweisen kann;
- Mercapto- (-SH) und Thioether-Gruppen (-SR"), wobei R" eine C₁₋₁₂-Alkyl-Gruppe, vorzugsweise C₂₋₁₀-Alkyl-Gruppe, besonders bevorzugt C₃₋₈-Alkyl-Gruppe ist, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan-(-CN) und Amid-Gruppen (-CONR₂'), substituiert sein kann, wobei R' die obigen Bedeutungen hat.
- R²: ist ausgewählt aus:
- linearen, verzweigten C₁₋₁₂-Alkyl-, C₂₋₁₂-Alkenyl-, C₂₋₁₂-Alkinyl-, cyclischen C₃₋₁₂-Alkyl-, C₄₋₁₂-Alkenyl-, C₄₋₁₂-Alkinyl-,oder C₅₋₁₈-Aryl-, C₇₋₁₉-Aralkyl oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN) und Amid-Gruppen (-CONR₂'), substituiert sein können, wobei R' die obigen Bedeutungen hat.
- R³: ist ausgewählt aus:
- Cyan- (-CN), Mercapto- (-SH), Thioether- (-SR"), Alkoxy- (-OR") und Acyl-Gruppen (-(C=O)-R"), wobei R" die obigen Bedeutungen hat;
- linearen, verzweigten C₁₋₁₂-Alkyl-, C₂₋₁₂-Alkenyl-, C₂₋₁₂-Alkinyl-, cyclischen C₃₋₁₂-Alkyl-, C₄₋₂-Alkenyl-, C₄₋₁₂-Alkinyl-, oder C₅₋₁₈-Aryl-, C₇₋₁₉-Aralkyl oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN) und Amid-Gruppen (-CONR₂'), substituiert sein kann, wobei R' die obigen Bedeutungen hat.
In einer alternativen erfindungsgemäßen Ausführungsform können R² und R³, R² und R¹ oder R¹ und R³ gemeinsam mit den Atomen, an die sie gebunden sind oder mit weiteren Atomen, ausgewählt aus N, O, P und S, einen vier- bis siebengliedrigen, vorzugsweise einen fünf- bis sechsgliedrigen Ring bilden, der mit -R', Halogen- (-X), Alkoxy- (-OR'), Thioether- oder Mercapto- (-SR'), Amino- (-NR'₂), Silyl- (-SiR'₃), Carboxyl- (-COOR'), Cyan- (-CN) und Amid-Gruppen (-CONR₂') substituiert sein kann, wobei R' die obigen Bedeutungen hat.
- R⁴: ist ausgewählt aus:
- Halogenatomen (X-);
- Cyan- (-CN), Mercapto- (-SH), Thioether- (-SR"), Alkoxy- (-OR") und Acyl-Gruppen (-(C=O)-R"), wobei R" die obigen Bedeutungen hat
- linearen, verzweigten C₁₋₁₂-Alkyl-, C₂₋₁₂-Alkenyl-, C₂₋₁₂-Alkinyl-, cyclischen C₃₋₁₂-Alkyl-, C₄₋₁₂-Alkenyl-, C₄₋₁₂-Alkinyl-,oder C₅₋₁₈-Aryl-, C₇₋₁₉-Aralkyl oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', Halogen- (-X), Alkoxy- (-OR'), Thioether- oder Mercapto-(-SR'), Amino- (-NR'₂), Silyl- (-SiR'₃), Carboxyl- (-COOR'), Cyan- (-CN) und Amid-Gruppen (-CONR₂'), substituiert sein kann, wobei R' die obigen Bedeutungen hat.
- R⁵: ist ausgewählt aus:
- Wasserstoff;
- Halogenatomen (X-);
- Cyan- (-CN), Mercapto- (-SH), Thioether- (-SR"), Alkoxy- (-OR") und Acyl-Gruppen (-(C=O)-R"), wobei R" die obigen Bedeutungen hat;
- linearen, verzweigten C₁₋₁₂-Alkyl-, C₂₋₁₂-Alkenyl-, C₂₋₁₂-Alkinyl-, cyclischen C₃₋₁₂-Alkyl-, C₄₋₁₂-Alkenyl-, C₄₋₁₂-Alkinyl-,oder C₅₋₁₈-Aryl-, C₇₋₁₉-Aralkyl oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', Halogen- (-X), Alkoxy- (-OR'), Thioether- oder Mercapto-(-SR'), Amino- (-NR'₂), Silyl- (-SiR'₃), Carboxyl- (-COOR'), Cyan- (-CN) und Amid-Gruppen (-CONR₂'), substituiert sein kann, wobei R' die obigen Bedeutungen hat.
- X¹: ist ausgewählt aus der Gruppe bestehend aus F, Cl, Br und I.
- X²: befindet sich entweder in 4- oder 5- Position der Phenyl-Gruppe und ist ausgewählt aus der Gruppe bestehend aus F, Cl, Br und I;

Im Falle einer 3,5-Dihalosubstitution sind X¹ und X² nicht gleichzeitig Cl.

Insbesondere soll das in WO-A-00/046 184 offenbarte N-Methyl-N-ethyl-N'-[4-(3,5-dichlor-phenoxy)-2,5-xylyl]-formamidin vom Schutzumfang ausgenommen sein.

In Formel (I) haben die Reste die im Folgenden definierten *bevorzugten* Bedeutungen. Die als *bevorzugt* getroffenen Definitionen gelten für alle Zwischenprodukte gleichermaßen:
- R¹: ist *vorzugsweise* ausgewählt aus der Gruppe bestehend aus Wasserstoff, einer Mercapto-Gruppe (-SH) oder C₁₋₈-Alkyl-Gruppen.
- R²: ist *vorzugsweise* ausgewählt aus linearen oder verzweigten, C₁₋₈-Alkyl-Gruppen,
- R³: ist *vorzugsweise* ausgewählt aus linearen, verzweigten und alicyclischen C₃₋₈-Alkyl-Gruppen.

In einer alternativen *vorzugsweisen* erfindungsgemäßen Ausführungsform können R² und R³ gemeinsam mit dem N-Atom, an das sie gebunden sind oder mit weiteren Atomen, ausgewählt aus N, O, P und S, einen fünf- bis sechsgliedrigen Ring bilden, der mit einer C₁₋₁₂-Alkyl-Gruppe, vorzugsweise C₂₋₁₀-Alkyl-Gruppe, besonders bevorzugt C₃₋₈-Alkyl-Gruppe substituiert sein kann.
- R⁴: ist *vorzugsweise* ausgewählt aus:
- Halogenatomen (-X);
- linearen oder verzweigten C₁₋₁₂-Alkyl-Gruppen und C₁₋₅-Haloalkylgruppen.
- R⁵: ist *vorzugsweise* ausgewählt aus:
- Halogenatomen (-X);
- linearen oder verzweigten C₁₋₁₂-Alkyl-Gruppen und C₁₋₅-Haloalkylgruppen.
- x¹: ist *vorzugsweise* ausgewählt aus der Gruppe bestehend aus F, Cl und Br.
- X²: befindet sich entweder in 4- oder 5- Position der Phenyl-Gruppe und ist *vorzugsweise* ausgewählt aus der Gruppe bestehend aus F, Cl und Br;

Im Falle einer 3,5-Dihalosubstitution sind X¹ und X² nicht gleichzeitig Cl.

In Formel (I) haben die Reste die im Folgenden definierten *besonders bevorzugten* Bedeutungen. Die als *besonders bevorzugt* getroffenen Definitionen gelten für alle Zwischenprodukte gleichermaßen:
- R¹: ist *besonders bevorzugt* ausgewählt aus
- Wasserstoff;
- Mercapto- (-SH), Methyl- und Ethyl-Gruppen.
- R²: ist *besonders bevorzugt* ausgewählt aus Methyl- und Ethyl-Gruppen.
- R³: ist *besonders bevorzugt* ausgewählt aus Ethyl- und Cyclopropyl-Gruppen.

In einer *besonders bevorzugten* alternativen erfindungsgemäßen Ausführungsform können R² und R³ mit dem N-Atom, an das sie geknüpft sind, einen Piperidyl- oder Pyrrolidyl-Ring bilden.
- R⁴: ist *besonders bevorzugt* ausgewählt aus Cl- und F-Atomen und -CF₃, -CF₂H und Methyl-Gruppen.
- R⁵: ist *besonders bevorzugt* ausgewählt aus Cl- und F-Atomen und -CF₃, -CF₂H und Methyl-Gruppen.
- X¹: ist *besonders bevorzugt* ausgewählt aus der Gruppe bestehend aus F, Cl und Br.
- X²: befindet sich *besonders bevorzugt* entweder in 4- oder 5- Position der Phenyl-Gruppe und ist ausgewählt aus der Gruppe bestehend aus F, Cl und Br.

Im Falle einer 3,5-Dihalosubstitution sind X¹ und X² nicht gleichzeitig Cl.

Je nach Art der oben definierten Substituenten weisen die Verbindungen der Formel (I) saure oder basische Eigenschaften auf und können mit anorganischen oder organischen Säuren oder mit Basen oder mit Metallionen Salze, gegebenenfalls auch innere Salze oder Addukte bilden.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Tragen die Verbindungen der Formel (I) Hydroxy, Carboxy oder andere, saure Eigenschaften induzierende Gruppen, so können diese Verbindungen mit Basen zu Salzen umgesetzt werden.

Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und teritäre Amine mit (C₁-C₄-)-Alkyl-Gruppen, Mono-, Di- und Trialkanolamine von (C₁-C₄)-Alkanolen, Cholin sowie Chlorcholin.

Tragen die Verbindungen der Formeln (I) Amino, Alkylamino oder andere, basische Eigenschaften induzierende Gruppen, so können diese Verbindungen mit Säuren zu Salzen umgesetzt werden.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄.

Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, Alkylsulfonsäuren (Sulfonsäuren mit geradkettigen oder verzweigten Alkyl-Gruppen mit 1 bis 20 Kohlenstoffatomen), Arylsulfonsäuren oder -disulfonsäuren (aromatische Reste wie Phenyl und Naphthyl welche ein oder zwei Sulfonsäuregruppen tragen), Alkylphosphon-säuren (Phosphonsäuren mit geradkettigen oder verzweigten Alkyl-Gruppen mit 1 bis 20 Kohlenstoffatomen), Arylphosphonsäuren oder - diphosphonsäuren (aromatische Reste wie Phenyl und Naphthyl welche ein oder zwei Phosphonsäure-Gruppen tragen), wobei die Alkyl- bzw. Aryl-Gruppen weitere Substituenten tragen können, z.B. p-Toluolsulfonsäure, Salicylsäure, p-Aminosalicylsäure, 2-Phenoxybenzoesäure, 2-Acetoxybenzoesäure etc..

Die so erhältlichen Salze weisen ebenfalls fungizide Eigenschaften auf.

Im Zusammenhang mit der vorliegenden Erfindung besonders bevorzugte Amidine sind ausgewählt aus der Gruppe bestehend aus:
N-Ethyl-N-methyl-N'-[4-(3,4-difluorphenoxy)-2,5-xylyl]formamidin; N-Methyl-N-propyl-N'-[4-(3,4-difluorphenoxy)-2,5-xylyl]formamidin;N-Cyclopropyl-N-methyl-N'-[4-(3,4-difluorphenoxy)-2,5-xylyl]formamidin; N,N-Diethyl-N'-[4-(3,4-difluorphenoxy)-2,5-xylyl]formamidin; N-Ethyl-N-propyl-N'-[4-(3,4-difluorphenoxy)-2,5-xylyl]formamidin;N-Pyrrolidinyl-N'-[4-(3,4-difluorphenoxy) -2,5-xylyl]formamidin; N-Piperidinyl-N'-[4-(3,4-difluorphenoxy)-2,5-xylyl]formamidin;N-(2,6-Dimethylmorphlinyl)-N'-[4-(3,4-difluorphenoxy)-2,5-xylyl]formamidin; N-Ethyl-N-methyl-N'-[4-(3,5-difluorphenoxy)-2,5-xylyl]formamidin; N-Methyl-N-propyl-N'-[4-(3,5-difluorphenoxy)-2,5-xylyl]formamidin; N-Cyclopropyl-N-methyl-N'-[4-(3,5-difluorphenoxy)-2,5-xylyl]formamidin; N,N-Diethyl-N'-[4-(3,5-difluorphenoxy)-2,5-xylyl]formamidin; N-Ethyl-N-propyl-N'-[4-(3,5-difluor-phenoxy)-2,5-xylyl]formamidin; N-Pyrrolidinyl-N'-[4-(3,5-difluorphenoxy)-2,5-xylyl]formamidin; N-Piperidinyl-N'-[4-(3,5-difluorphenoxy)-2,5-xylyl]formamidin; N-(2,6-Dimethylmorphlinyl)-N'-[4-(3,5-difluorphenoxy)-2,5-xylyl]formamidin; N-Ethyl-N-methyl-N'-[4-(3-chlor-4-fluorphenoxy)-2,5-xylyl]formamidin; N-Methyl-N-propyl-N'-[4-(3-chlor-4-fluorphenoxy)-2,5-xylyl]formamidin; N-Cyclopropyl-N-methyl-N'-[4-(3-chlor-4-fluorphenoxy)-2,5-xylyl]formamidin; N,N-Diethyl-N'-[4-(3-chlor-4-fluorphenoxy)-2,5-xylyl]formamidin; N-Ethyl-N-propyl-N'-[4-(3-chlor-4-fluorphenoxy)-2,5-xylyl]formamidin; N-Pyrrolidinyl-N'-[4-(3-chlor-4-fluorphenoxy)-2,5-xylyl]formamidin; N-Piperidinyl-N'-[4-(3-chlor-4-fluorphenoxy)-2,5-xylyl]formamidin; N-(2,6-Dimethylmorpholinyl)-N'-[4-(3-chlor-4-fluorphenoxy)-2,5-xylyl]formamidin; N-Ethyl-N-methyl-N'-[4-(3-chlor-5-fluor-phenoxy)-2,5-xylyl]formamidin; N-Methyl-N-propyl-N'-[4-(3-chlor-5-fluorphenoxy)-2,5-xylyl]formamidin; N-Cyclopropyl-N-methyl-N'-[4-(3-chlor-5-fluorphenoxy)-2,5-xylyl]formamidin; N,N-Diethyl-N'-[4-(3-chlor-5-fluorphenoxy)-2,5-xylyl]formamidin; N-Ethyl-N-propyl-N'-[4-(3-chlor-5-fluorphenoxy)-2,5-xylyl]formamidin; N-Pyrrolidinyl-N'-[4-(3-chlor-5-fluorphenoxy)-2,5-xylyl]formamidin; N-Piperidinyl-N'-[4-(3-chlor-5-fluorphenoxy)-2,5-xylyl]formamidin; N-(2,6-Dimethylmorphlinyl)-N'-[4-(3-chlor-5-fluorphenoxy)-2,5-xylyl]formamidin; N-Ethyl-N-methyl-N'-[4-(3-brom-4-fluorphenoxy)-2,5-xylyl]formamidin; N-Methyl-N-propyl-N'-[4-(3-brom-4-fluor-phenoxy)-2,5-xylyl]formamidin; N-Cyclopropyl-N-methyl-N'-[4-(3-brom-4-fluorphenoxy)-2,5-xylyl]formamidin; N,N-Diethyl-N'-[4-(3-brom-4-fluorphenoxy)-2,5-xylyl]formamidin; N-Ethyl-N-propyl-N'-[4-(3-brom-4-fluorphenoxy)-2,5-xylyl]formamidin; N-Pyrrolidinyl-N'-[4-(3-brom-4-fluorphenoxy)-2,5-xylyl]formamidin; N-Piperidinyl-N'-[4-(3-brom-4-fluorphenoxy)-2,5-xylyl] formamidin; N-(2,6-Dimethylmorpholinyl)-N'-[4-(3-brom-4-fluorphenoxy)-2,5-xylyl]formamidin; N-Ethyl-N-methyl-N'-[4-(3-brom-5-fluorphenoxy)-2,5-xylyl]formamidin; N-Methyl-N-propyl-N'-[4-(3-brom-5-fluorphenoxy)-2,5-xylyl]formamidin; N-Cyclopropyl-N-methyl-N'-[4-(3-brom-5-fluorphenoxy)-2,5-xylyl]formamidin; N,N-Diethyl-N'-[4-(3-brom-5-fluorphenoxy)-2,5-xylyl]-formamidin; N-Ethyl-N-propyl-N'-[4-(3-brom-5-fluorphenoxy)-2,5-xylyl]formamidin; N-Pyrrolidinyl-N'-[4-(3-brom-5-fluorphenoxy)-2,5-xylyl]formamidin; N-Piperidinyl-N'-[4-(3-brom-5-fluorphenoxy)-2,5-xylyl]formamidin; N-(2,6-Dimethylmorphlinyl)-N'-[4-(3-brom-5-fluorphenoxy)-2,5-xylyl]formamidin; N-Ethyl-N-methyl-N'-[4-(3-iod-4-fluorphenoxy)-2,5-xylyl]formamidin; N-Methyl-N-propyl-N'-[4-(3-iod-4-fluorphenoxy)-2,5-xylyl]formamidin; N-Cyclopropyl-N-methyl-N'-[4-(3-iod-4-fluorphenoxy)-2,5-xylyl]formamidin; N,N-Diethyl-N'-[4-(3-iod-4-fluorphenoxy)-2,5-xylyl]formamidin; N-Ethyl-N-propyl-N'-[4-(3-iod-4-fluorphenoxy)-2,5-xylyl]formamidin; N-Pyrrolidinyl-N'-[4-(3-iod-4-fluorphenoxy)-2,5-xylyl]formamidin; N-Piperidinyl-N'-[4-(3-iod-4-fluorphenoxy)-2,5-xylyl]formamidin; N-(2,6-Dimethylmorphlinyl)-N'-[4-(3-iod-4-fluorphenoxy)-2,5-xylyl]formamidin; N-Ethyl-N-methyl-N'-[4-(3-iod-5-fluorphenoxy)-2,5-xylyl]formamidin; N-Methyl-N-propyl-N'-[4-(3-iod-5-fluorphenoxy)-2,5-xylyl]formamidin; N-Cyclopropyl-N-methyl-N'-[4-(3-iod-5-fluorphenoxy)-2,5-xylyl]formamidin; N,N-Diethyl-N'-[4-(3-iod-5-fluorphenoxy)-2,5-xylyl]formamidin; N-Ethyl-N-propyl-N'-[4-(3-iod-5-fluorphenoxy)-2,5-xylyl]formamidin; N-Pyrrolidinyl-N'-[4-(3-iod-5-fluorphenoxy)-2,5-xylyl]formamidin; N-Piperidinyl-N'-[4-(3-iod-5-fluorphenoxy)-2,5-xylyl]formamidin;N-(2,6-Dimethylmorphlinyl)-N'-[4-(3-iod-5-fluorphenoxy)-2,5-xylyl]formamidin; N-Ethyl-N-methyl-N'-[4-(3-fluor-4-chlorphenoxy)-2,5-xylyl]formamidin; N-Methyl-N-propyl-N'-[4-(3-fluor-4-chlorphenoxy)-2,5-xylyl]formamidin; N-Cyclopropyl-N-methyl-N'-[4-(3-fluor-4-chlorphenoxy)-2,5-xylyl]formamidin; N,N-Diethyl-N'-[4-(3-fluor-4-chlorphenoxy)-2,5-xylyl]formamidin; N-Ethyl-N-propyl-N'-[4-(3-fluor-4-chlorphenoxy)-2,5-xylyl]formamidin; N-Pyrrolidinyl-N'-[4-(3-fluor-4-chlorphenoxy)-2,5-xylyl]formamidin; N-Piperidinyl-N'-[4-(3-fluor-4-chlorphenoxy)-2,5-xylyl]formamidin; N-(2,6-Dimethylmorphlinyl)-N'-[4-(3-fluor-4-chlorphenoxy)-2,5-xylyl]formamidin; N-Ethyl-N-methyl-N'-[4-(3,4-dichlorphenoxy)-2,5-xylyl]formamidin; N-Methyl-N-propyl-N'-[4-(3,4-dichlorphenoxy)-2,5-xylyl]formamidin; N-Cyclopropyl-N-methyl-N'-[4-(3,4-dichlorphenoxy)-2,5-xylyl]formamidin; N,N-Diethyl-N'-[4-(3,4-dichlorphenoxy)-2,5-xylyl]-formamidin; N-Ethyl-N-propyl-N'-[4-(3,4-dichlorphenoxy)-2,5-xylyl]formamidin; N-Pyrrolidinyl-N'-[4-(3,4-dichlorphenoxy)-2,5-xylyl]formamidin; N-Piperidinyl-N'-[4-(3,4-dichlorphenoxy)-2,5-xylyl]formamidin; N-(2,6-Dimethylmorphlinyl)-N'-[4-(3,4-dichlorphenoxy)-2,5-xylyl]formamidin; N-Ethyl-N-methyl-N'-[4-(3-brom-4-chlorphenoxy)-2,5-xylyl]formamidin; N-Methyl-N-propyl-N'-[4-(3-brom-4-chlorphenoxy)-2,5-xylyl]formamidin; N-Cyclopropyl-N-methyl-N'-[4-(3-brom-4-chlorphenoxy)-2,5-xylyl]formamidin; N,N-Diethyl-N'-[4-(3-brom-4-chlorphenoxy)-2,5-xylyl] formamidin; N-Ethyl-N-propyl-N'-[4-(3-brom-4-chlorphenoxy)-2,5-xylyl]formamidin; N-Pyrrolidinyl-N'-[4-(3-brom-4-chlorphenoxy)-2,5-xylyl]formamidin; N-Piperidinyl-N'-[4-(3-brom-4-chlorphenoxy)-2,5-xylyl]formamidin; N-(2,6-Dimethylmorphlinyl)-N'-[4-(3-brom-4-chlorphenoxy)-2,5-xylyl]formamidin; N-Ethyl-N-methyl-N'-[4-(3-brom-5-chlorphenoxy)-2,5-xylyl]formamidin; N-Methyl-N-propyl-N'-[4-(3-brom-5-chlorphenoxy)-2,5-xylyl]-formamidin; N-Cyclopropyl-N-methyl-N'-[4-(3-brom-5-chlorphenoxy)-2,5-xylyl]formamidin; N,N-Diethyl-N'-[4-(3-brom-5-chlor-phenoxy)-2,5-xylyl]formamidin; N-Ethyl-N-propyl-N'-[4-(3-brom-5-chlorphenoxy)-2,5-xylyl] formamidin; N-Pyrrolidinyl-N'-[4-(3-brom-5-chlorphenoxy)-2,5-xylyl]formamidin; N-Piperidinyl-N'-[4-(3-brom-5-chlorphenoxy)-2,5-xylyl]formamidin; N-(2,6-Dimethylmorphlinyl)-N'-[4-(3-brom-5-chlorphenoxy)-2,5-xylyl]formamidin; N-Ethyl-N-methyl-N'-[4-(3-iod-4-chlorphenoxy)-2,5-xylyl]formamidin; N-Methyl-N-propyl-N'-[4-(3-iod-4-chlorphenoxy)-2,5-xylyl]formamidin; N-Cyclopropyl-N-methyl-N'-[4-(3-iod-4-chlorphenoxy)-2,5-xylyl]formamidin; N,N-Diethyl-N'-[4-(3-iod-4-chlorphenoxy)-2,5-xylyl]formamidin; N-Ethyl-N-propyl-N'-[4-(3-iod-4-chlorphenoxy)-2,5-xylyl]formamidin; N-Pyrrolidinyl-N'-[4-(3-iod-4-chlorphenoxy)-2,5-xylyl]formamidin; N-Piperidinyl-N'-[4-(3-iod-4-chlorphenoxy)-2,5-xylyl]formamidin; N-(2,6-Dimethylmorphlinyl)-N'-[4-(3-iod-4-chlorphenoxy)-2,5-xylyl]formamidin; N-Ethyl-N-methyl-N'-[4-(3-iod-5-chlorphenoxy)-2,5-xylyl]formamidin; N-Methyl-N-propyl-N'-[4-(3-iod-5-chlorphenoxy)-2,5-xylyl]formamidin; N-Cyclopropyl-N-methyl-N'-[4-(3-iod-5-chlorphenoxy)-2,5-xylyl]formamidin; N,N-Diethyl-N'-[4-(3-iod-5-chlorphenoxy)-2,5-xylyl]formamidin; N-Ethyl-N-propyl-N'-[4-(3-iod-5-chlorphenoxy)-2,5-xylyl]formamidin; N-Pyrrolidmyl-N'-[4-(3-iod-5-chlorphenoxy)-2,5-xylyl]formamidin; N-Piperidinyl-N'-[4-(3-iod-5-chlorphenoxy)-2,5-xylyl]formamidin; N-(2,6-Dimethylmorphlinyl)-N'-[4-(3-iod-5-chlorphenoxy)-2,5-xylyl]formamidin; N-Ethyl-N-methyl-N'-[4-(3-fluor-4-bromphenoxy)-2,5-xylyl]formamidin; N-Methyl-N-propyl-N'-[4-(3-fluor-4-bromphenoxy)-2,5-xylyl]formamidin; N-Cyclopropyl-N-methyl-N'-[4-(3-fluor-4-bromphenoxy)-2,5-xylyl]formamidin; N,N-Diethyl-N'-[4-(3-fluor-4-bromphenoxy)-2,5-xylyl]formamidin; N-Ethyl-N-propyl-N'-[4-(3-fluor-4-brom-phenoxy)-2,5-xylyl]formamidin; N-Pyrrolidinyl-N'-[4-(3-fluor-4-bromphenoxy)-2,5-xylyl]-formamidin; N-Piperidinyl-N'-[4-(3-fluor-4-bromphenoxy)-2,5-xylyl]formamidin; N-(2,6-Dimethylmorphlinyl)-N'-[4-(3-fluor-4-bromphenoxy)-2,5-xylyl]formamidin; N-Ethyl-N-methyl-N'-[4-(3-chloro-4-bromphenoxy)-2,5-xylyl]formamidin; N-Methyl-N-propyl-N'-[4-(3-chloro-4-brom-phenoxy)-2,5-xylyl]formamidin; N-Cyclopropyl-N-methyl-N'-[4-(3-chloro-4-bromphenoxy)-2,5-xylyl]formamidin; N,N-Diethyl-N'-[4-(3-chloro-4-bromphenoxy)-2,5-xylyl]formamidin; N-Ethyl-N-propyl-N'-[4-(3-chloro-4-bromphenoxy)-2,5-xylyl]formamidin; N-Pyrrolidinyl-N'-[4-(3-chloro-4-bromphenoxy)-2,5-xylyl]formamidin; N-Piperidinyl-N'-[4-(3-chloro-4-bromphenoxy)-2,5-xylyl]formamidin; N-(2,6-Dimethylmorphlinyl)-N'-[4-(3-chloro-4-bromphenoxy)-2,5-xylyl]formamidin;N-Ethyl-N-methyl-N'-[4-(3,4-dibromphenoxy)-2,5-xylyl]formamidin; N-Methyl-N-propyl-N'-[4-(3,4-dibromphenoxy)-2,5-xylyl]formamidin; N-Cyclopropyl-N-methyl-N'-[4-(3,4-dibrom-phenoxy)-2,5-xylyl]formamidin; N,N-Diethyl-N'-[4-(3,4-dibromphenoxy)-2,5-xylyl]formamidin; N-Ethyl-N-propyl-N'-[4-(3,4-dibromphenoxy)-2,5-xylyl]formamidin; N-Pyrrolidinyl-N'-[4-(3,4-dibromphenoxy)-2,5-xylyl]formamidin; N-Piperidinyl-N'-[4-(3,4-dibromphenoxy)-2,5-xylyl]formamidin; N -(2, 6-Dimethylmorphlinyl)-N' -[4-(3, 4-dibromphenoxy)-2,5-xylyl]formamidin; N-Ethyl-N-methyl-N'-[4-(3,5-dibromphenoxy)-2,5-xylyl]formamidin; N-Methyl-N-propyl-N'-[4-(3,5-dibrom-phenoxy)-2,5-xylyl]formamidin; N-Cyclopropyl-N-methyl-N'-[4-(3,5-dibromphenoxy)-2,5-xylyl]formamidin; N,N-Diethyl-N'-[4-(3,5-dibromphenoxy)-2,5-xylyl]formamidin; N-Ethyl-N-propyl-N'-[4-(3,5-dibromphenoxy)-2,5-xylyl]formamidin; N-Pyrrolidinyl-N'-[4-(3,5-dibromphenoxy)-2,5-xylyl]formamidin; N-Piperidinyl-N'-[4-(3,5-dibromphenoxy)-2,5-xylyl]formamidin; N-(2,6-Dimethylmorphlinyl)-N'-[4-(3,5-dibromphenoxy)-2,5-xylyl]formamidin; N-Ethyl-N-methyl-N'-[4-(3-iod-4-bromphenoxy)-2,5-xylyl]formamidin; N-Methyl-N-propyl-N'-[4-(3-iod-4-brom-phenoxy)-2,5-xylyl]formamidin; N-Cyclopropyl-N-methyl-N'-[4-(3-iod-4-bromphenoxy)-2,5-xylyl]formamidin; N,N-Diethyl-N'-[4-(3-iod-4-bromphenoxy)-2,5-xylyl]formamidin; N-Ethyl-N-propyl-N'-[4-(3-iod-4-bromphenoxy)-2,5-xylyl]formamidin; N-Pyrrolidinyl-N'-[4-(3-iod-4-bromphenoxy)-2,5-xylyl]formamidin; N-Piperidinyl-N'-[4-(3-iod-4-bromphenoxy)-2,5-xylyl] formamidin; N-(2,6-Dimethylmorphlinyl)-N'-[4-(3-iod-4-bromphenoxy)-2,5-xylyl]formamidin; N-Ethyl-N-methyl-N'-[4-(3-iod-5-bromphenoxy)-2,5-xylyl]formamidin; N-Methyl-N-propyl-N'-[4-(3-iod-5-bromphenoxy)-2,5-xylyl]formamidin; N-Cyclopropyl-N-methyl-N'-[4-(3-iod-5-bromphenoxy)-2,5-xylyl]formamidin; N,N-Diethyl-N'-[4-(3-iod-5-bromphenoxy)-2,5-xylyl]formamidin; N-Ethyl-N-propyl-N'-[4-(3-iod-5-bromphenoxy)-2,5-xylyl]formamidin; N-Pyrrolidinyl-N'-[4-(3-iod-5-bromphenoxy)-2,5-xylyl]formamidin; N-Piperidinyl-N'-[4-(3-iod-5-bromphenoxy)-2,5-xylyl]-formamidin; N-(2,6-Dimethylmorphlinyl)-N'-[4-(3-iod-5-bromphenoxy)-2,5-xylyl]formamidin; N-Ethyl-N-methyl-N'-[4-(3-fluor-4-iodphenoxy)-2,5-xylyl]formamidin; N-Methyl-N-propyl-N'-[4-(3-fluor-4-iodphenoxy)-2,5-xylyl]formamidin;N-Cyclopropyl-N-methyl-N'-[4-(3-fluor-4-iodphenoxy)-2,5-xylyl]formamidin; N,N-Diethyl-N'-[4-(3-fluor-4-iodphenoxy)-2,5-xylyl]formamidin; N-Ethyl-N-propyl-N'-[4-(3-fluor-4-iodphenoxy)-2,5-xylyl]formamidin; N-Pyrrolidinyl-N'-[4-(3-fluor-4-iodphenoxy)-2,5-xylyl]formamidin; N-Piperidinyl-N'-[4-(3-fluor-4-iodphenoxy)-2,5-xylyl] formamidin; N-(2,6-Dimethylmorphlinyl)-N'-[4-(3-fluor-4-iodphenoxy)-2,5-xylyl]formamidin; N-Ethyl-N-methyl-N'-[4-(3-chloro-4-iodphenoxy)-2,5-xylyl]formamidin; N-Methyl-N-propyl-N'-[4-(3-chloro-4-iodphenoxy)-2,5-xylyl]formamidin; N-Cyclopropyl-N-methyl-N'-[4-(3-chloro-4-iodphenoxy)-2,5-xylyl]formamidin; N,N-Diethyl-N'-[4-(3-chloro-4-iodphenoxy)-2,5-xylyl]formamidin; N-Ethyl-N-propyl-N'-[4-(3-chlorö-4-iodphenoxy)-2,5-xylyl]formamidin; N-Pyrrolidinyl-N'-[4-(3-chloro-4-iodphenoxy)-2,5-xylyl]formamidin; N-Piperidinyl-N'-[4-(3-chloro-4-iodphenoxy)-2,5-xylyl]formamidin; N-(2,6-Dimethylmorphlinyl)-N'-[4-(3-chloro-4-iodphenoxy)-2,5-xylyl]-formamidin; N-Ethyl-N-methyl-N'-[4-(3-brom-4-iodphenoxy)-2,5-xylyl]formamidin; N-Methyl-N-propyl-N'-[4-(3-brom-4-iodphenoxy)-2,5-xylyl]formamidin; N-Cyclopropyl-N-methyl-N'-[4-(3-brom-4-iodphenoxy)-2,5-xylyl]formamidin; N,N-Diethyl-N'-[4-(3-brom-4-iodphenoxy)-2,5-xylyl]formamidin; N-Ethyl-N-propyl-N'-[4-(3-brom-4-iodphenoxy)-2,5-xylyl]formamidin; N-Pyrrolidinyl-N'-[4-(3-brom-4-iodphenoxy)-2,5-xylyl]formamidin; N-Piperidinyl-N'-[4-(3-brom-4-iodphenoxy)-2,5-xylyl]formamidin; N-(2,6-Dimethylmorphlinyl)-N'-[4-(3-brom-4-iodphenoxy)-2,5-xylyl]formamidin; N-Ethyl-N-methyl-N'-[4-(3,4-düodphenoxy)-2,5-xylyl]formamidin; N-Methyl-N-propyl-N'-[4-(3,4-diiodphenoxy)-2,5-xylyl]formamidin; N-Cyclopropyl-N-methyl-N'-[4-(3,4-diiod- phenoxy)-2,5-xylyl]formamidin, N,N-Diethyl-N'-[4-(3,4-diiodphenoxy)-2,5-xylyl]formamidin; N-Ethyl-N-propyl-N'-[4-(3,4-diiodphenoxy)-2,5-xylyl]formamidin; N-Pyrrolidinyl-N'-[4-(3,4-diiod-phenoxy)-2,5-xylyl]formamidin; N-Piperidinyl-N'-[4-(3,4-diiodphenoxy)-2,5-xylyl]formamidin; N-(2,6-Dimethylmorphlinyl)-N'-[4-(3,4-diiodphenoxy)-2,5-xylyl]formamidin; N-Ethyl-N-methyl-N'-[4-(3,5-düodphenoxy)-2,5-xylyl]formamidin; N-Methyl-N-propyl-N'-[4-(3,5-diiodphenoxy)-2,5-xylyl]formamidin; N-Cyclopropyl-N-methyl-N'-[4-(3,5-diiodphenoxy)-2,5-xylyl]formamidin; N,N-Diethyl-N'-[4-(3,5-diiodphenoxy)-2,5-xylyl]formamidin; N-Ethyl-N-propyl-N'-[4-(3,5-diiod-phenoxy)-2,5-xylyl]formamidin; N-Pyrrolidinyl-N'-[4-(3,5-diiodphenoxy)-2,5-xylyl]formamidin; N-Piperidinyl-N'-[4-(3,5-diiodphenoxy)-2,5-xylyl]formamidin; N-(2,6-Dimethylmorphlinyl)-N'-[4-(3,5-diiodphenoxy)-2,5-xylyl]formamidin.

### Herstellung der erfindungsgemäßen Amidine

Die erfindungsgemäßen Dihalogenphenoxyphenylamidin können durch das im folgenden Schema (I) dargestellte Verfahren erhalten werden:

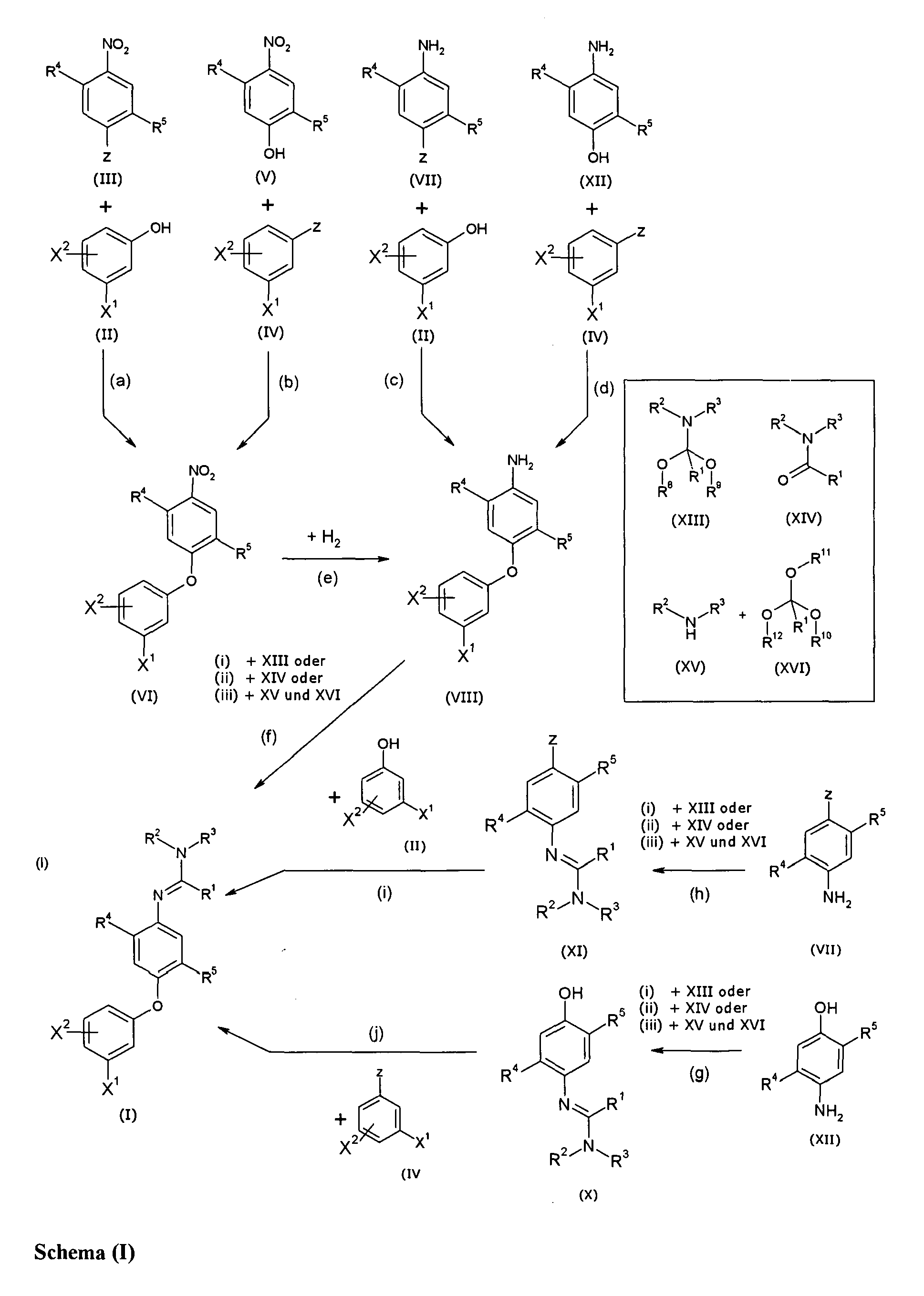

### Schritt (a)

In einer erfindungsgemäßen Ausführungsform werden Nitrobenzolderivate der Formel (III) mit Dihalogenphenolen der Formel (II) oder den aus diesen gebildeten Phenolaten gemäß dem nachfolgenden Reaktionsschema zu Nitrophenylethern der Formel (VI) umgesetzt:

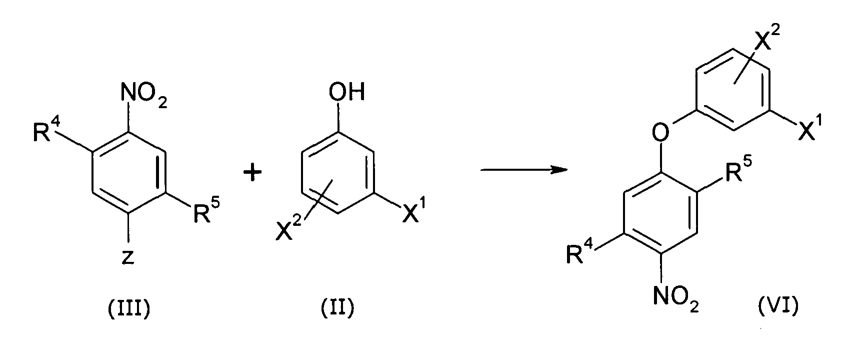

Als Austrittsgruppe (Z) sind alle Substituenten geeignet, die bei den vorherrschenden Reaktionsbedingungen eine ausreichende Nucleofugizität aufweisen. Beispielhaft seien Halogene, Triflat, Mesylat, Tosylat oder SO₂Me als geeignete Austrittsgruppen genannt.

Die Reaktion erfolgt vorzugsweise in Gegenwart einer Base.

Geeignete Basen sind organische und anorganische Basen, die üblicherweise in solchen Reaktionen verwendet werden. Vorzugsweise werden Basen verwendet, die beispielhaft ausgewählt sind aus der Gruppe bestehend aus Hydriden, Hydroxiden, Amiden, Alkoholaten, Acetaten, Fluoriden, Phosphaten, Carbonaten und Hydrogencarbonaten von Alkali- oder Erdalkalimetallen. Besonders bevorzugt sind dabei Natriumamid, Natriumhydrid, Lithiumdiisopropylamid, Natriummethanolat, Kalium-*tert*-butanolat, Natriumhydroxid, Kaliumhydroxid, Natriumacetat, Natriumphosphat, Kaliumphosphat, Kaliumfluorid, Caesiumfluorid, Natriumcarbonat, Kaliumcarbonat, Kaliumhydrogencarbonat, Natriumhydrogencarbonat und Caesiumcarbonat. Außerdem sind tertiäre Amine, wie z.B. Trimethylamin, Triethylamin, Tributylamin, N,N-Dimethylanilin, N,N-Dimethylbenzylamin, Pyridin, N-Methylpiperidin, N-Methylpyrolidon, N,N-Dimethylaminopyridin, Diazabicyclooctan (DABCO), Diazabicyclononen (DBN) und Diazabicycloundecen (DBU).

Gegebenenfalls kann ein Katalysator, der ausgewählt ist aus der Gruppe bestehend aus Palladium, Kupfer und deren Salzen oder Komplexen eingesetzt werden.

Die Reaktion des Nitrobenzolderivats mit dem Phenol kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln.

Bevorzugt werden aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzonitril; Amide, wie beispielsweise N,N-Dimethylformamid (DMF), N,N-Dimethylacetamid, N-Methylformanilid, N-Methylpyrrolidon (NMP) oder Hexamethylenphosphorsäuretriamid; oder Mischungen dieser mit Wasser sowie reines Wasser.

Die Reaktion kann im Vakuum, bei Normaldruck oder unter Überdruck und bei Temperaturen von - 20 bis 200 °C durchgeführt werden, vorzugsweise erfolgt die Reaktion bei Normaldruck und Temperaturen von 50 bis 150 °C.

### Schritt (b)

In einer alternativen erfindungsgemäßen Ausführungsform werden Nitrophenolderivate der Formel (V) oder die daraus gebildeten Phenolate mit Dihalogenphenylderivaten der Formel (IV) gemäß dem nachfolgenden Reaktionsschema zu Nitrophenylethern der Formel (VI) umgesetzt:

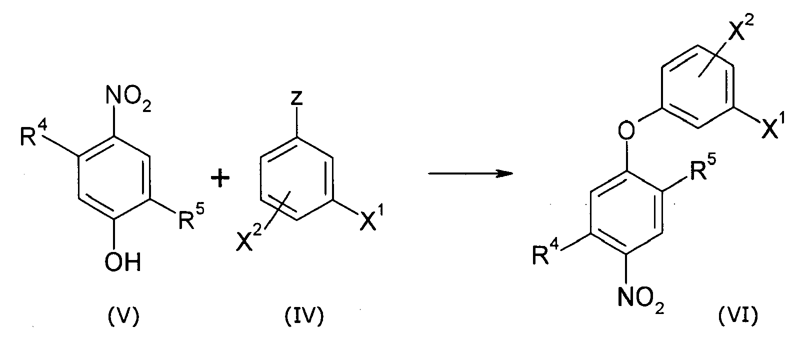

Im Hinblick auf Reaktionsbedingungen, Lösungsmittel, Katalysatoren und geeignete Austrittsgruppen (Z) sei auf Schritt (a) verwiesen.

### Schritt (c)

In einer weiteren alternativen erfindungsgemäßen Ausführungsform werden Aniline der Formel (VII) mit Dihalogenphenolen der Formel (II) oder den aus diesen gebildeten Phenolaten gemäß dem nachfolgenden Reaktionsschema zu Aminophenylethern der Formel (VIII) umgesetzt:

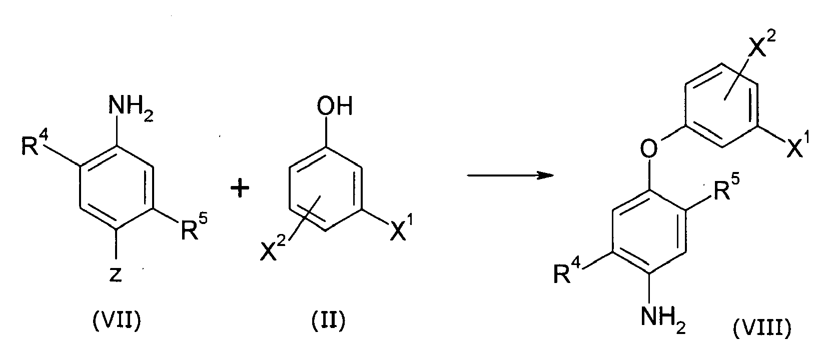

Im Hinblick auf Reaktionsbedingungen, Lösungsmittel, Katalysatoren und geeignete Austrittsgruppen (Z) sei auf Schritt (a) verwiesen.

### Schritt (d)

In einer weiteren alternativen erfindungsgemäßen Ausführungsform werden Aminophenole der Formel (XII) mit Dihalogenphenylderivaten der Formel (IV) gemäß dem nachfolgenden Reaktionsschema zu Aminophenylethern der Formel (VIII) umgesetzt:

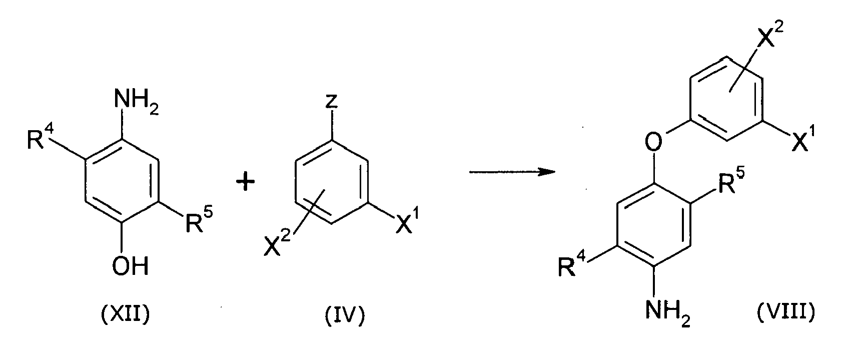

Im Hinblick auf Reaktionsbedingungen, Lösungsmittel, Katalysatoren und geeignete Austrittsgruppen sei auf Schritt (c) verwiesen.

### Schritt (e)

Die in den Schritten (a) und (b) erhaltenen Nitrophenylether der Formel (VI) können gemäß dem nachfolgenden Reaktionsschema zu den Anilinethern der Formel (VIII) reduziert werden:

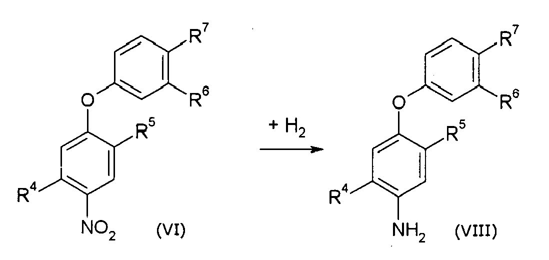

Die Reduktion gemäß Schritt (e) kann durch sämtliche im Stand der Technik zur Reduktion von Nitro-Gruppen beschriebenen Methoden erfolgen.

Vorzugsweise erfolgt die Reduktion mit Zinnchlorid in konzentrierter Salzsäure wie in WO 0046184 beschrieben. Alternativ kann die Reduktion aber auch mit Wasserstoffgas, ggf. in Gegenwart geeigneter Hydrierkatalysatoren, wie z.B. Raney Nickel oder Pd/C erfolgen. Die Reaktionsbedingungen sind im Stand der Technik vorbeschrieben und dem Fachmann geläufig.

Wird die Reduktion in der Flüssigphase durchgeführt, so sollte die Reaktion in einem gegenüber den vorherrschenden Reaktionsbedingungen inerten Lösungsmittel erfolgen. Ein solches ist beispielsweise Toluol.

### Schritt (f)

Die Umsetzung der Anilinethern der Formel (VIII) zu den erfindungsgemäßen Amidinen der Formel (I) gemäß Schritt (f) kann, wie zuvor in Schema (I) dargestellt nach unterschiedlichen alternativen Methoden unter Verwendung von
(i) Aminoacetalen der Formel (XIII) oder
(ii) Amiden der Formel (XIV) oder
(iii) Aminen der Formel (XV) in Gegenwart von Orthoestern der Formel (XVI)
gemäß dem nachfolgenden Reaktionsschema erfolgen:

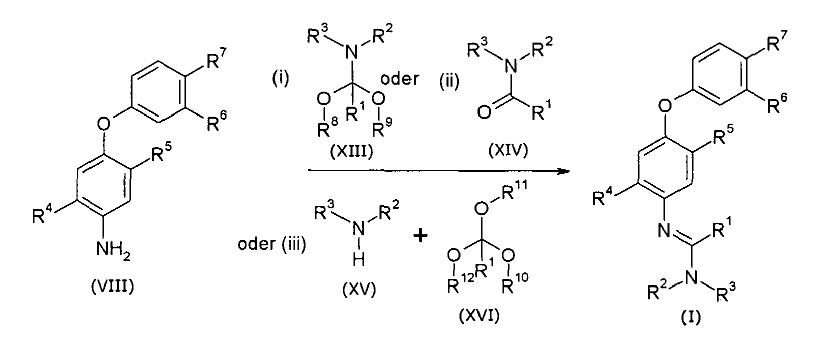

Die einzelnen alternativen Ausführungsformen (i) bis (iii) des erfindungsgemäßen Verfahrens sollen infolge kurz erläutert werden:
(i) Gemäß einer erfindungsgemäßen Ausführungsform, die im Schema (I) als Schritt (i) dargestellt ist, werden die Anilinether der Formel (VIII) mit Aminoacetalen der Formel (XIII), in der R² und R³ wie zuvor beschrieben definiert sind und R⁸ und R⁹ ausgewählt sind aus C₁₋₈-Alkyl-Gruppen, vorzugsweise aus C₂₋₆-Alkyl-Gruppen, besonders bevorzugt aus C₃₋₅-Alkyl-Gruppen und gemeinsam einen fünf- oder sechsgliedrigen carbocyclischen Ring bilden können, zu den erfindungsgemäßen Phenoxyamidinen der Formel (I) umgesetzt.
   Die Aminoacetale der Formel (XIII) sind aus den in JACS, 65, 1566 (1943) beschriebenen Formamiden durch Umsetzung mit Alkylierungsreagenzien, wie z.B. Dimethylsulfat, erhältlich.
   Die Reaktion gemäß Schritt (i) erfolgt vorzugsweise in Gegenwart einer Säure.
   Geeignete Säuren sind beispielsweise ausgewählt aus der Gruppe bestehend aus organischen und anorganischen Säuren, wobei p-Toluolsulfonsäure, Methansulfonsäure, Salzsäure (gasförmig, wässrig oder in organischer Lösung) oder Schwefelsäure.
(ii) In einer alternativen erfindungsgemäßen Ausführungsform, die im Schema (I) als Schritt (ii) dargestellt ist, werden die Anilinether der Formel (VIII) mit Amiden der Formel (XIV), in der die Reste R¹ bis R³ wie zuvor definiert sind, zu den erfindungsgemäßen Phenoxyamidinen umgesetzt.
   Die Reaktion gemäß Schritt (ii) erfolgt ggf. in Gegenwart eines Halogenierungsmittels. Geeignete Halogenierungsmittel sind beispielsweise ausgewählt aus der Gruppe bestehend aus PCl₅, PCl₃, POCl₃ oder SOCl₂.
   Zudem kann die Reaktion alternativ in Gegenwart eines Kondensationsmittels erfolgen.
   Geeignete Kondensationsmittel sind solche, die üblicherweise zur Knüpfung von Amidbindungen verwendet werden, beispielhaft seien Säurehalogenidbildner wie z.B. Phosgen, Phosphortribromid, Phosphortrichlorid, Phosphorpentachlorid, Phosphortrichloridoxid oder Thionylchlorid; Anhydridbildner wie z.B. Chlorformiat, Methylchlorformiat, Isopropylchlorformiat, Isobutylchlorformiat oder Methansulfonylchlorid; Carbodiimine wie z.B. N,N'-Dicyclohexylcarbodiimin (DCC) oder andere übliche Kondensationsmittel wie z.B. Phosphorpentoxid, Polyphosphorsäure, N,N'-Carbodiimidazol, 2-Ethoxy-N-ethoxycarbonyl-1,2-dihydroquinolin (EEDQ), Triphenylphosphin/Tetrachlormethan oder Bromtripyrrolidinophosphoniumhexafluorophosphat genannt.
   Die Reaktion gemäß Schritt (ii) erfolgt vorzugsweise in einem Lösungsmittel, welches ausgewählt ist aus den üblichen, bei den vorherrschenden Reaktionsbedingungen inerten Lösungsmitteln. Bevorzugt werden aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzonitril; Amide, wie beispielsweise N,N-Dimethylformamid (DMF), N,N-Dimethylacetamid, N-Methylformanilid, N-Methylpyrrolidon (NMP) oder Hexamethylenphosphorsäuretriamid; Ester, wie beispielsweise Methyl- oder Ethylacetat; Sulfoxide, wie beispielsweise Dimethylsulfoxid (DMSO); Sulfone, wie beispielsweise Sulfolan; Alkohole, wie beispielsweise Methanol, Ethanol, n- oder iso-Propanol, n-, iso-, sec- oder tert-Butanol, Ethandiol, Propan-1,2-diol, Ethoxiethanol, Methoxyethanol, Diethylenglycolmonomethylether, Diethylenglycolmonoethylether oder Mischungen dieser verwendet.
(iii) Gemäß einer weiteren alternativen erfindungsgemäßen Ausführungsform, die in Schema (I) als Schritt (iii) dargestellt ist, werden die Anilinether der Formel (VIII) mit Aminen der Formel (XV), in der die Reste R² und R³ wie zuvor definiert sind in Gegenwart von Orthoestern der Formel (XVI), in der R¹ wie zuvor definiert ist und R¹⁰ bis R¹² unabhängig voneinander ausgewählt sind aus C₁₋₈-Alkyl-Gruppen, vorzugsweise aus C₂₋₆-Alkyl-Gruppen, besonders bevorzugt aus C₃₋₅-Alkyl-Gruppen, zu den erfindungsgemäßen dihalogensubstituierten Phenoxyamidinen umgesetzt.
   Die Reaktion gemäß Schritt (iii) erfolgt vorzugsweise in einem Lösungsmittel, welches ausgewählt ist aus den üblichen, bei den vorherrschenden Reaktionsbedingungen inerten Lösungsmitteln. Bevorzugt werden aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie z.B. Chlorbenzol, Dichlorbenzol, Dichlormethan, Chloroform, Tetrachlorkohlenstoff, Dichlorethan oder Trichlorethan; Ether, wie beispielsweise Diethylether, Diisopropylether, Methyl-tert-butylether (MTBE), Methyl-tert-amylether, Dioxan, Tetrahydrofuran, 1,2-Dimethoxyethan, 1,2-Diethoxyethan oder Anisol; Nitrile, wie beispielsweise Acetonitril, Propionitril, n- oder iso-Butyronitril oder Benzonitril; Amide, wie beispielsweise N,N-Dimethylformamid (DMF), N,N-Dimethylacetamid, N-Methylformanilid, N-Methylpyrrolidon (NMP) oder Hexamethylenphosphorsäuretriamid; Ester, wie beispielsweise Methyl- oder Ethylacetat; Sulfoxide, wie beispielsweise Dimethylsulfoxid (DMSO); Sulfone, wie beispielsweise Sulfolan; Alkohole, wie beispielsweise Methanol, Ethanol, n- oder iso-Propanol, n-, iso-, sec- oder tert-Butanol, Ethandiol, Propan-1,2-diol, Ethoxiethanol, Methoxyethanol, Diethylenglycolmonomethylether, Diethylenglycolmonoethylether; oder Mischungen dieser mit Wasser sowie reines Wasser verwendet.

### Schritt (g)

In einer alternativen erfindungsgemäßen Ausführungsform können bereits die Aminophenole der Formel (XII)
(i) mit Aminoacetalen der Formel (XIII) oder
(ii) mit Amiden der Formel (XIV) oder
(iii) mit Aminen der Formel (XV) in Gegenwart von Orthoestern der Formel (XVI)
gemäß dem nachfolgenden Reaktionsschema zu Amidinen der Formel (X) umgesetzt werden:

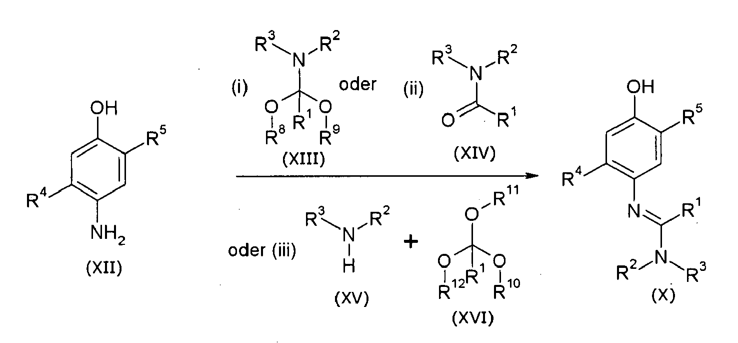

Hinsichtlich der Reaktionsbedingungen, Lösungsmittel, Katalysatoren sei auf Schritt (f) verwiesen.

Die weitere Umsetzung der Amidine der Formel (X) zu den erfindungsgemäßen Zielmolekülen der Formel (I) kann beispielsweise wie in Schritt (j) beschrieben erfolgen.

### Schritt (h)

In einer alternativen erfindungsgemäßen Ausführungsform können die Aminophenylderivate der Formel (VII)
(i) mit Aminoacetalen der Formel (XIII) oder
(ii) mit Amiden der Formel (XIV) oder
(iii) mit Aminen der Formel (XV) in Gegenwart von Orthoestern der Formel (XVI)
gemäß dem nachfolgenden Reaktionsschema zu Amidinen der Formel (XI) umgesetzt werden:

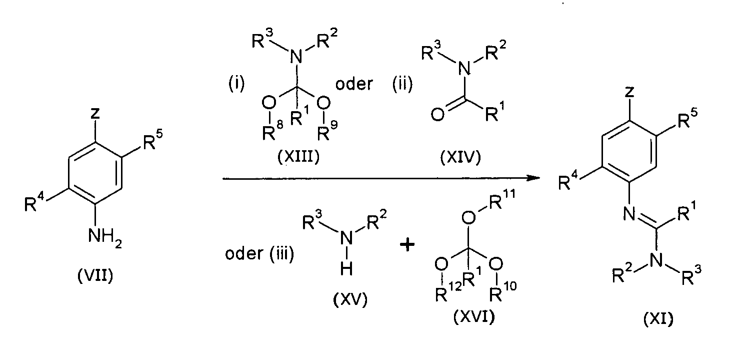

Hinsichtlich der Reaktionsbedingungen, Lösungsmittel, Katalysatoren sei auf Schritt (f) verwiesen.

Die weitere Umsetzung der Amidine der Formel (XI) zu den erfindungsgemäßen Zielmolekülen der Formel (I) kann beispielsweise wie in Schritt (i) beschrieben erfolgen.

### Schritt (i)

Gemäß einer weiteren erfindungsgemäßen Ausführungsform können die aus Schritt (h) erhältlichen Amidine der Formel (XI) mit Dihalogenphenolen der Formel (II) oder den aus diesen gebildeten Phenolaten zu den erfindungsgemäßen Zielmolekülen der Formel (I) gemäß dem nachfolgenden Reaktionsschema umgesetzt werden:

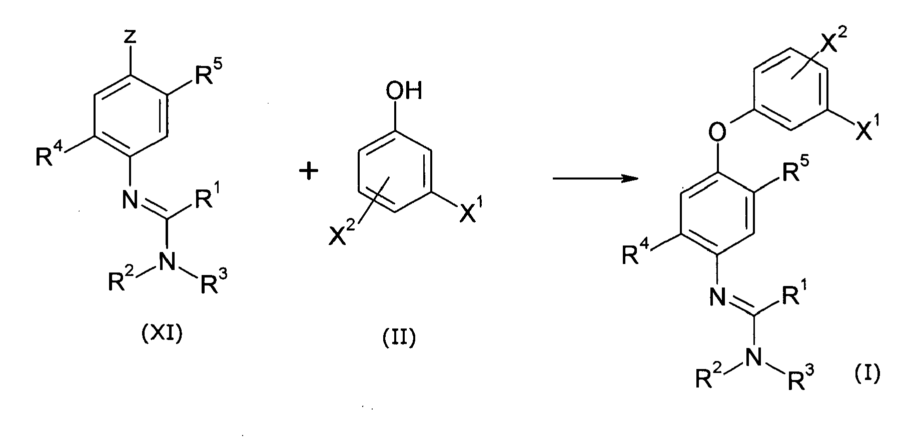

Hinsichtlich der Reaktionsbedingungen, Austrittsgruppen (Z), Lösungsmittel und Katalysatoren sei auf Schritt (a) verwiesen.

### Schritt (j)

Gemäß einer weiteren erfindungsgemäßen Ausführungsform können die aus Schritt (g) erhältlichen Amidine der Formel (X) mit Dihalogenphenylderivaten der Formel (IV) zu den erfindungsgemäßen Zielmolekülen der Formel (I) gemäß dem nachfolgenden Reaktionsschema umgesetzt werden:

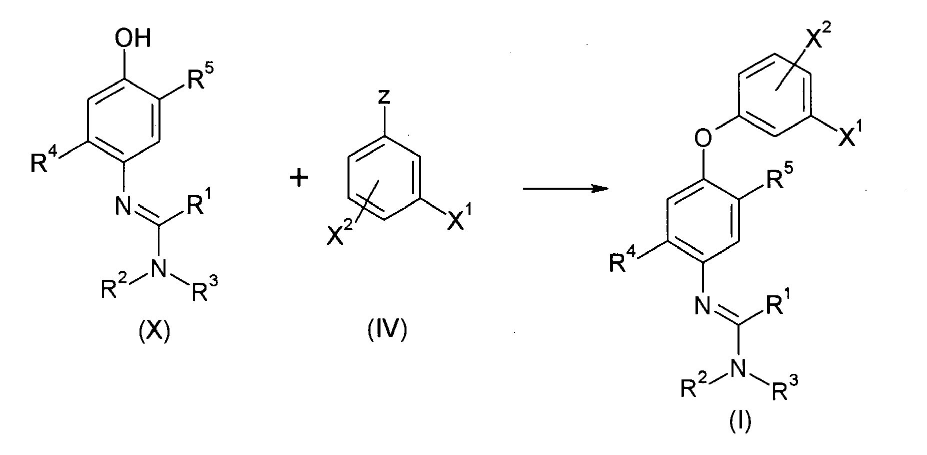

Hinsichtlich der Reaktionsbedingungen, Austrittsgruppen (Z), Lösungsmittel und Katalysatoren sei auf Schritt (b) verwiesen.

Im Zusammenhang mit den erfindungsgemäßen Verfahren zur Herstellung der Amidine der Formel (I) sind die folgenden Kombinationen von Reaktionsschritten als Vorteilhaft anzusehen: Schritte (a), (e) und (f); Schritte (b), (e) und (f); Schritte (c) und (f); Schritte (d) und (f); Schritte (h) und (i) und/oder Schritte (g) und (j).

Die Herstellung der erfindungsgemäßen Phenoxyamidine erfolgt ggf ohne zwischenzeitliche Isolierung der Zwischenprodukte.

Die abschließende Reinigung der Phenoxyamidinen kann durch übliche Reinigungsverfahren erfolgen. Vorzugsweise erfolgt die Reinigung durch Kristallisation.

### Bekämpfung, von unerwünschten Mikroorganismen

Die erfindungsgemäßen Amidine weisen eine starke mikrobizide Wirkung auf und können zur Bekämpfung von unerwünschten Mikroorganismen, wie Fungi und Bakterien, im Pflanzenschutz und im Materialschutz eingesetzt werden.

### Pflanzenschutz

Fungizide lassen sich im Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Erkrankungen, hervorgerufen durch Erreger des Echten Mehltaus wie z.B.
Blumeria-Arten, wie beispielsweise Blumeria graminis;
Podosphaera-Arten, wie beispielsweise Podosphaera leucotricha;
Sphaerotheca-Arten, wie beispielsweise Sphaerotheca fuliginea;
Uncinula-Arten, wie beispielsweise Uncinula necator;

Erkrankungen, hervorgerufen durch Erreger von Rostkrankheiten wie z.B.
Gymnosporangium-Arten, wie beispielsweise Gymnosporangium sabinae
Hemileia-Arten, wie beispielsweise Hemileia vastatrix;
Phakopsora-Arten, wie beispielsweise Phakopsora pachyrhizi und Phakopsora meibomiae;
Puccinia-Arten, wie beispielsweise Puccinia recondita;
Uromyces-Arten, wie beispielsweise Uromyces appendiculatus;

Erkrankungen, hervorgerufen durch Erreger der Gruppe der Oomyceten wie z.B.
Bremia-Arten, wie beispielsweise Bremia lactucae;
Peronospora-Arten, wie beispielsweise Peronospora pisi oder P. brassicae;
Phytophthora-Arten, wie beispielsweise Phytophthora infestans;
Plasmopara-Arten, wie beispielsweise Plasmopara viticola;
Pseudoperonospora-Arten, wie beispielsweise Pseudoperonospora humuli oder
Pseudoperonospora cubensis;
Pythium-Arten, wie beispielsweise Pythium ultimum;

Blattfleckenkrankheiten und Blattwelken, hervorgerufen durch z.B.
Alternaria-Arten, wie beispielsweise Alternaria solani;
Cercospora-Arten, wie beispielsweise Cercospora beticola;
Cladosporium-Arten, wie beispielsweise Cladosporium cucumerinum;
Cochliobolus-Arten, wie beispielsweise Cochliobolus sativus
(Konidienform: Drechslera, Syn: Helminthosporium);
Colletotrichum-Arten, wie beispielsweise Colletotrichum lindemuthanium;
Cycloconium-Arten, wie beispielsweise Cycloconium oleaginum;
Diaporthe-Arten, wie beispielsweise Diaporthe citri;
Elsinoe-Arten, wie beispielsweise Elsinoe fawcettii;
Gloeosporium-Arten, wie beispielsweise Gloeosporium laeticolor;
Glomerella-Arten, wie beispielsweise Glomerella cingulata;
Guignardia-Arten, wie beispielsweise Guignardia bidwelli;
Leptosphaeria-Arten, wie beispielsweise Leptosphaeria maculans;
Magnaporthe-Arten, wie beispielsweise Magnaporthe grisea;
Mycosphaerella-Arten, wie beispielsweise Mycosphaerella graminicola und Mycosphaerella fijiensis;
Phaeosphaeria-Arten, wie beispielsweise Phaeosphaeria nodorum;
Pyrenophora-Arten, wie beispielsweise Pyrenophora teres;
Ramularia-Arten, wie beispielsweise Ramularia collo-cygni;
Rhynchosporium-Arten, wie beispielsweise Rhynchosporium secalis;
Septoria-Arten, wie beispielsweise Septoria apii;
Typhula-Arten, wie beispielsweise Typhula incarnata;
Venturia-Arten, wie beispielsweise Venturia inaequalis;

Wurzel- und Stengelkrankheiten, hervorgerufen durch z.B.
Corticium-Arten, wie beispielsweise Corticium graminearum;
Fusarium-Arten, wie beispielsweise Fusarium oxysporum;
Gaeumannomyces-Arten, wie beispielsweise Gaeumannomyces graminis;
Rhizoctonia-Arten, wie beispielsweise Rhizoctonia solani;
Tapesia-Arten, wie beispielsweise Tapesia acuformis;
Thielaviopsis-Arten, wie beispielsweise Thielaviopsis basicola;
Ähren- und Rispenerkrankungen (inklusive Maiskolben), hervorgerufen durch z.B.
Alternaria-Arten, wie beispielsweise Alternaria spp.;
Aspergillus-Arten, wie beispielsweise Aspergillus flavus;
Cladosporium-Arten, wie beispielsweise Cladosporium cladosporioides;
Claviceps-Arten, wie beispielsweise Claviceps purpurea;
Fusarium-Arten, wie beispielsweise Fusarium culmorum;
Gibberella-Arten, wie beispielsweise Gibberella zeae;
Monographella-Arten, wie beispielsweise Monographella nivalis;

Erkrankungen, hervorgerufen durch Brandpilze wie z.B.
Sphacelotheca-Arten, wie beispielsweise Sphacelotheca reiliana;
Tilletia-Arten, wie beispielsweise Tilletia caries;
Urocystis-Arten, wie beispielsweise Urocystis occulta;
Ustilago-Arten, wie beispielsweise Ustilago nuda;

Fruchtfäule hervorgerufen durch z.B.
Aspergillus-Arten, wie beispielsweise Aspergillus flavus;
Botrytis-Arten, wie beispielsweise Botrytis cinerea;
Penicillium-Arten, wie beispielsweise Penicillium expansum und Penicillium purpurogenum;
Sclerotinia-Arten, wie beispielsweise Sclerotinia sclerotiorum;
Verticilium-Arten, wie beispielsweise Verticilium alboatrum;
Samen- und bodenbürtige Fäulen und Welken, sowie Sämlingserkrankungen, hervorgerufen durch z.B.
Fusarium-Arten, wie beispielsweise Fusarium culmorum;
Phytophthora Arten, wie beispielsweise Phytophthora cactorum;
Pythium-Arten, wie beispielsweise Pythium ultimum;
Rhizoctonia-Arten, wie beispielsweise Rhizoctonia solani;
Sclerotium-Arten, wie beispielsweise Sclerotium rolfsii;

Krebserkrankungen, Gallen und Hexenbesen, hervorgerufen durch z.B.
Nectria-Arten, wie beispielsweise Nectria galligena;

Welkeerkrankungen hervorgerufen durch z.B.
Monilinia-Arten, wie beispielsweise Monilinia laxa;

Deformationen von Blättern, Blüten und Früchten, hervorgerufen durch z.B.
Taphrina-Arten, wie beispielsweise Taphrina deformans;

Degenerationserkrankungen holziger pflanzen, hervorgerufen durch z.B.
Esca-Arten, wie beispielsweise Phaeomoniella chlamydospora und Phaeoacremonium aleophilum und Fomitiporia mediterranea;
Blüten- und Samenerkrankungen, hervorgerufen durch z.B.
Botrytis-Arten, wie beispielsweise Botrytis cinerea;
Erkrankungen von Pflanzenknollen, hervorgerufen durch z.B.
Rhizoctonia-Arten, wie beispielsweise Rhizoctonia solani;
Helminthosporium-Arten, wie beispielsweise Helminthosporium solani;
Erkrankungen, hervorgerufen durch bakterielle Erreger wie z.B.
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Bevorzugt können die folgenden Krankheiten von Soja-Bohnen bekämpft werden:
Pilzkrankheiten an Blättern, Stängeln, Schoten und Samen verursacht durch z.B.
Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi), Scab (Sphaceloma glycines), Stemphylium Leaf Blight (Stemphylium botryosum), Target Spot (Corynespora cassiicola)
Pilzkrankheiten an Wurzeln und der Stängelbasis verursacht durch z.B.
Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irregulare, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraums nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie z.B. gegen Puccinia-Arten und von Krankheiten im Wein-, Obst- und Gemüseanbau, wie z.B. gegen Botrytis-, Venturia- oder Alternaria-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

### Mycotoxine

Darüber hinaus kann durch die erfindungsgemäße Behandlung der Mykotoxingehalt im Erntegut und den daraus hergestellten Nahrungs- und Futtermitteln verringert werden. Besonders, aber nicht ausschließlich sind hierbei folgende Mykotoxine zu nennen: Deoxynivalenol (DON), Nivalenol, 15-Ac-DON, 3-Ac-DON, T2- und HT2- Toxin, Fumonisine, Zearalenon, Moniliformin, Fusarin, Diaceotoxyscirpenol (DAS), Beauvericin, Enniatin, Fusaroproliferin, Fusarenol, Ochratoxine, Patulin, Mutterkomalkaloide und Aflatoxine, die beispielsweise von den folgenden Pilzen verursacht werden können: Fusarium spec., wie Fusarium acuminatum, F. avenaceum, F. crookwellense, F. culmorum, F. graminearum (Gibberella zeae), F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides u.a. sowie auch von Aspergillus spec., Penicillium spec., Claviceps purpurea, Stachybotrys spec. u.a.

### Materialschutz

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

### Formulierungen

Die vorliegende Erfindung betrifft ein Mittel zum Bekämpfen unerwünschter Mikroorganismen, umfassend wenigstens eines der erfindungsgemäßen Phenoxyamidin.

Die erfindungsgemäßen Phenoxyamidin können dazu in Abhängigkeit von ihren jeweiligen physikalischen und/ oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/ oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen infrage: z.B. gebrochene und fraktionierte natürliche Gesteine wie Calcit, Bims, Marmor, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen infrage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die zuvor beschriebenen Formulierungen können in einem erfindungsgemäßen Verfahren zum Bekämpfen unerwünschter Mikroorganismen verwendet werden, bei dem die erfindungsgemäßen Phenoxyamidin auf die Mikroorganismen und/oder in deren Lebensraum ausgebracht werden.

### Saatgutbehandlung

Ein weiterer Aspekt der vorliegenden Erfindung betrifft insbesondere ein Saatgut, welches mit wenigstens einem der erfindungsgemäßen Phenoxyamidin behandelt ist.

Die erfindungsgemäßen Saatgüter finden Anwendung in Verfahren zum Schutz von Saatgut vor unerwünschten Mikroorganismen. Bei diesen wird ein mit wenigstens einem erfindungsgemäßen Phenoxyamidin behandelten Saatgut. Verwendet.

Die Bekämpfung von phytopathogenen Pilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von phytopathogenen Pilzen, indem das Saatgut mit einem erfindungsgemäßen Mittel behandelt wird.

Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen.

Weiterhin bezieht sich die Erfindung auf Saatgut, welches zum Schutz vor phytopathogenen Pilzen mit einem erfindungsgemäßen Mittel behandelt wurde.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Mischungen insbesondere auch bei transgenem Saatgut eingesetzt werden können.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffkombinationen können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Düsobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidön, Polyvinylacetat, Polyvinylalkohol und Tylose.

Als Gibberelline, die in den erfindungsgemäß verwendbaren Beizmittel-Formulierungen enthalten sein können, kommen vorzugsweise Stoffe der Formel (XVII) 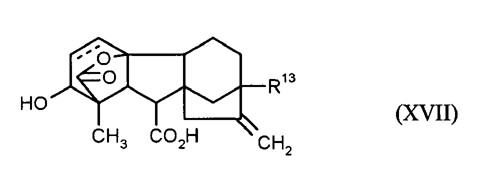 in welcher
- R¹³: für Wasserstoff oder Hydroxy steht und
die gestrichelte Linie andeutet, dass an der Stelle des Ringes entweder eine C-C-Einfachbindung oder eine C=C-Doppelbindung enthalten ist,
in Betracht.

Als Beispiele für Gibberelline der Formel (XVII) seien genannt: 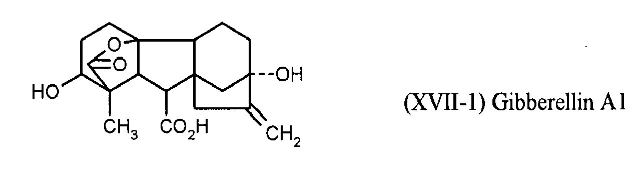 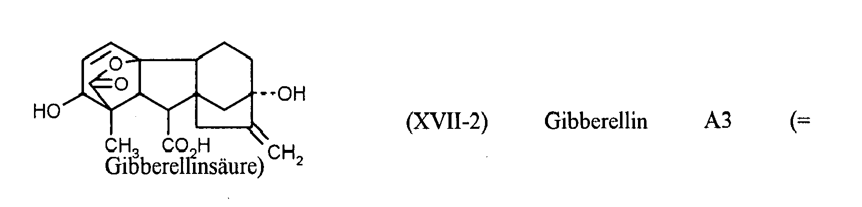 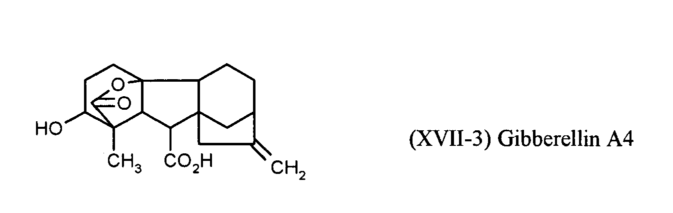 und

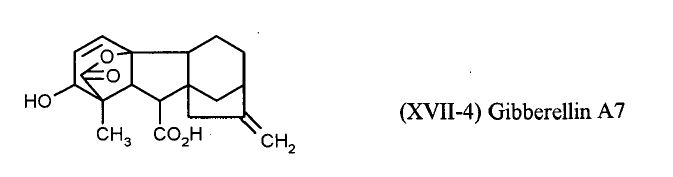

Besonders bevorzugt ist die Gibberellinsäure der Formel (XVII-2).

Die Gibberelline der Formel (XVII) sind bekannt (vgl. R. Wegler "Chemie der Pflanzenschutz- und Schädlingsbekämpfungsmittel", Band 2, Springer Verlag, Berlin-Heidelberg-New York, 1970, Seiten 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

### Mischung mit bekannten Fungiziden, Bakteriziden, Akariziden, Nematiziden oder Insektiziden

Die erfindungsgemäßen Phenoxyamidin können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Auch eine Mischung mit anderen bekannten Wirkstoffen, wie Herbiziden oder mit Düngemitteln und Wachstumsregulatoren, Safener bzw. Semiochemicals ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Dihalogenphenoxyphenylamidine können daher sowohl für medizinische als auch für nicht-medizinische Anwendungen eingesetzt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Es kann auch das Saatgut der Pflanzen behandelt werden.

Beim Einsatz der erfindungsgemäßen Phenoxyamidin als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im Allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausfühnmgsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante ©-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, weiter entwickeltes Wurzelsystem, höhere Beständigkeit der Pflanzenart bzw. Pflanzensorte, gesteigertes Wachstum der Schösslinge, höhere Pflanzenvitalität, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, größere Früchte, höhere Pflanzengröße, grünere Blattfarbe, frühere Blüte, höhere Qualität und/oder höherer Ernährungswert der Ernteprodukte, höhere Zuckerkonzentration in den Früchten, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Tabak, Raps sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, z.B. Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Herstellung und die Verwendung der erfindungsgemäßen Wirkstoffe soll anhand der folgenden Beispiele näher erläutert werden, ohne jedoch auf diese beschränkt zu sein.

### Herstellungsbeispiele

### Beispiel 1: N-Ethyl-N-methyl-N'-[4-(3,4-dichlorphenoxy)-2,5-dimethylphenyl]formamidin (30)

200 mg (0.71 mmol) 4-(3,4-Dichlorphenoxy)-2,5-dimethylanilin werden in 5 ml Trimethylorthoformiat 4 h refluxiert. Die Reaktionsmischung wird auf Raumtemperatur abgekühlt und im Vakuum einrotiert. Der Rückstand wird in 50 ml Dichlormethan aufgenommen und mit 84 mg (1.42 mmol) N-Ethyl-N-methylamin versetzt. Nach 16 h Rühren bei Raumtemperatur warden Lösungsmittel und nicht umgesetztes Amin im Vakuum entfernt, das Produkt säulenchromatographisch aufgereinigt (230 mg, 88% Reinheit, 80% Ausbeute, log P (pH 2,3) = 2.48).

### Synthese der Ausgangsverbindungen:

### 4-(3,4-Dichlorphenoxy)-2,5-dimethylanilin

Eine Lösung von 18,00 g (57,6 mmol) 4-(3,4-Dichlorphenoxy)-2,5-dimethylnitrobenzol in 40 ml Dioxan und 40 ml Salzsäure wird mit 39,0 g (173,0 mmol) Zinn(II)chlorid-Dihydrat bei Raumtemperatur versetzt und anschliessend für 2 h refluxiert. Man kühlt auf Raumtemperatur ab, neutralisiert mit NaHCO₃, extrahiert mehrfach mit Dichlormethan, trocknet über Na₂SO₄, filtriert und entfernt das Lösungsmittel im Vakuum. (18,3 g, 89 % Reinheit, 99,5 % Ausbeute, log P (pH 2,3) = 3,51).

### 4-(3,4-Dichlorphenoxy)-2,5-dimethylnitrobenzol

10.0 g (61,3 mmol) 3,4-Dichlorphenol, 10,4 g (55,8 mmol) 4-Chlor-2,5-dimethylnitrobenzol und 10,0 g (72,5 mmol) Kaliumcarbonat werden in 200 ml N,N-Dimethylformamid 6h bei 100°C gerührt, auf Eis gegeben, 15 min. bei 0°C gerührt und anschliessend filtriert. Der Feststoff wird mit Wasser und Hexan gewaschen und im Vakuum getrocknet. (18.0 g, 98.3 % Reinheit, 98.0 % Ausbeute, log P (pH 2.3) = 5.19).

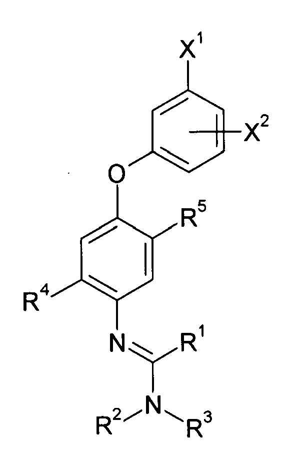

| **Tabelle I** | | | | | | | | | |
|---|---|---|---|---|---|---|---|---|---|
| **Nr.** | **R¹** | **R²** | **R³** | **R⁴** | **R⁵** | **X¹** | **X²** | **logP sauer** | **logP neutr.** |
| 1 | H | Me | Et | Me | Me | 3-F | 4-Cl | | |
| 2 | H | Me | Et | Me | Me | 3-F | 4-F | 2,37 | 6,2 |
| 3 | H | Me | Et | Me | Me | 3-F | 5-Br | 2,29 | 5,8 |
| 4 | H | Me | Et | Me | Me | 3-Br | 5-Br | 2,5 | 6 |
| 5 | H | Me | i-Pr | Me | Me | 3-Br | 5-Br | 2,62 | 6,4 |
| 6 | H | Me | Et | Me | Me | 3-Br | 5-Cl | 2,56 | 6,4 |
| 7 | H | Me | i-Pr | Me | Me | 3-Br | 5-Cl | 2,7 | 6,8 |
| 8 | H | Me | Et | Me | Me | 3-C1 | 5-F | 2,21 | 5,6 |
| 9 | H | -(CH₂)₅- | | Me | Me | 3-Cl | 5-F | 2,42 | 6,3 |
| 10 | H | -(CH₂)₄- | | Me | Me | 3-C1 | 5-F | 2,26 | 5,6 |
| 11 | H | Me | n-Pr | Me | Me | 3-Cl | 5-F | 2,39 | 6,1 |
| 12 | H | -(CH₂)₅- | | Me | Me | 3-Br | 5-F | 2,55 | 6,5 |
| 13 | H | -(CH₂)₄- | | Me | Me | 3-Br | 5-F | 2,37 | 5,7 |
| 14 | H | Me | n-Pr | Me | Me | 3-Br | 5-F | 2,37 | 7,4 |
| 15 | H | Me | Et | Me | Me | 4-Cl | 3-Br | 2,23 | 6,1 |
| 16 | H | -(CH₂)₅- | | Me | Me | 4-Cl | 3-Br | 2,66 | 6,9 |
| 17 | H | Me | n-Pr | Me | Me | 4-Cl | 3-Br | 2,64 | 6,6 |
| 18 | H | -(CH₂)₄- | | Me | Me | 4-Cl | 3-Br | 2,56 | 6,6 |
| 19 | H | Me | Et | Me | Me | 3-I | 5-F | 2,4 | |
| 20 | H | Me | n-Pr | Me | Me | 3-I | 5-F | 2,61 | 6,6 |
| 21 | H | -(CH₂)₅- | | Me | Me | 3-I | 5-F | 2,6 | 6,8 |
| 22 | H | -(CH₂)₄- | | Me | Me | 3-I | 5-F | 2,46 | 6,6 |
| 23 | H | Me | Et | Me | Me | 3-Br | 4-F | 2,29 | 5,7 |
| 24 | H | Me | i-Pr | Me | Me | 3-I | 4-C1 | 2,4 | 6,2 |
| 25 | H | Me | Et | Me | Me | 3-I | 4-C1 | 2,29 | |
| 26 | H | -(CH₂)₅- | | Me | Me | 3-I | 4-C1 | 2,52 | |
| 27 | H | Me | Et | Me | Me | 3-I | 4-Br | 2,67 | 6,1 |
| 28 | H | Me | n-Pr | Me | Me | 3-Br | 5-F | 2,91 | 6,9 |
| 29 | H | -CH₂CH(CH₃)-O-CH(CH₃)-CH₂- | | Me | Me | 3-Br | 5-F | 2,59 | 5,8 |
| 30 | H | Me | Et | Me | Me | 3-Cl | 3-Cl | | |

### Verwendungsbeispiele

### Beispiel 1: Puccinia-Test (Weizen) / protektiv

| | |
|---|---|
| Lösungsmittel: | 50 Gewichtsteile *N,N Dimethylacetamid* |
| | |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Puccinia recondita besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Temperatur von ca. 20°C und einer relativen Luftfeuchtigkeit von 80 % aufgestellt, um die Entwicklung von Rostpusteln zu begünstigen.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die erfindungsgemäßen Verbindungen der Formeln **3, 4, 5, 6, 7, 8, 9, 11, 12, 13, 14, 15, 1 6, 19, 21, 23, 24** und **25** (siehe Tabelle **I)** bei einer Konzentration an Wirkstoff von 1000 ppm einen Wirkungsgrad von 70% oder mehr.

### Beispiel 2: Erysiphe-Test (Gerste) / protektiv

| | | |
|---|---|---|
| Lösungsmittel: | 49 Gewichtsteile | N,N-Dimethylformamid |
| | | |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Getreidepflanzen mit der Wirkstoffzubereitung in der angegebenen Aufwandmenge. 1 Tag nach der Behandlung werden die Pflanzen mit Sporen von Erysiphe graminis f. sp. hordei inokuliert. Anschließend werden die Pflanzen in einem Gewächshaus bei 70 % relativer Luftfeuchtigkeit und einer Temperatur von 18°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die erfindungsgemäßen Verbindungen der Formeln **3, 4, 5, 6, 7, 8, 9, 11, 12, 13 und 14** (siehe Tabelle I) bei einer Konzentration an Wirkstoff von 500 ppm einen Wirkungsgrad von 70% oder mehr.

### Beispiel 3: Leptosphaeria nodorum - Test (Weizen) / protektiv

| | | |
|---|---|---|
| Lösungsmittel: | 49 Gewichtsteile | N,N-Dimethylformamid |
| | | |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Weizenpflanzen mit der Wirkstoffzubereitung in der angegebenen Aufwandmenge. 1 Tag nach der Behandlung werden die Pflanzen mit einer wäßrigen Sporensuspension von *Leptosphaeria nodorum* inokuliert und verbleiben dann 48h bei 100% rel. Luftfeuchte und 20°C. Anschließend werden die Pflanzen in einem Gewächshaus bei 80 % rel. Luftfeuchtigkeit und einer Temperatur von 22°C aufgestellt.

12-14 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

In diesem Test zeigen die erfindungsgemäßen Verbindungen der Formeln 3, 15, 21, 24, 25, 26 und 27 (siehe Tabelle I) bei einer Konzentration an Wirkstoff von 500 ppm einen Wirkungsgrad von 70% oder mehr.

### Beispiel 4: Podosphaera-Test (Apfel) / protektiv

| | |
|---|---|
| Lösungsmittel: | 24,5 Gewichtsteile Aceton |
| | |
| | 24,5 Gewichtsteile Dimethylacetamid |
| | |
| Emulgator: | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension des Apfelmehltauerregers ***Podosphaera leucotricha*** inokuliert. Die Pflanzen werden dann im Gewächshaus bei ca. 23°C und einer relativen Luftfeuchtigkeit von ca. 70% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen der der Formeln **3**, **4**, **6**, **7**, **9**, **11**, **24** und **25** (siehe Tabelle I) bei einer Konzentration an Wirkstoff von 100 ppm einen Wirkungsgrad von 70% oder mehr.

### Beispiel 5: Sphaerotheca-Test (Gurke) / protektiv

| | |
|---|---|
| Lösungsmittel: | 24,5 Gewichtsteile Aceton |
| | |
| | 24,5 Gewichtsteile Dimethylacetamid |
| | |
| Emulgator: | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Sphaerotheca fuliginea*** inokuliert. Die Pflanzen werden dann bei ca. 23°C und einer relativen Luftfeuchtigkeit von ca. 70% im Gewächshaus aufgestellt.

7 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen der Formeln **3**, **4**, **6**, **7**, **9**, **11**, **23** und **24** (siehe Tabelle I) bei einer Konzentration an Wirkstoff von 100ppm einen Wirkungsgrad von 70% oder mehr.

### Beispiel 6: Uromyces - Test (Bohne) / protektiv

| | |
|---|---|
| Lösungsmittel : | 24,5 Gewichtsteile Aceton |
| | |
| | 24,5 Gewichtsteile Dimethylacetamid |
| | |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension des Bohnenrosterregers ***Uromyces appendiculatus*** inokuliert und verbleiben dann 1 Tag bei ca. 20°C und 100% relativer Luftfeuchtigkeit in einer Inkubations-kabine.

Die Pflanzen werden dann im Gewächshaus bei ca. 21°C und einer relativen Luftfeuchtigkeit von ca. 90% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die erfindungsgemäßen Verbindungen der Formeln **3**, **7**, **23**, **24** und **34** (siehe Tabelle I) bei einer Konzentration an Wirkstoff von 10ppm einen Wirkungsgrad von 70% oder mehr.

## Patentansprüche

1. Dihalogenphenoxyphenylamidine der Formel (I) 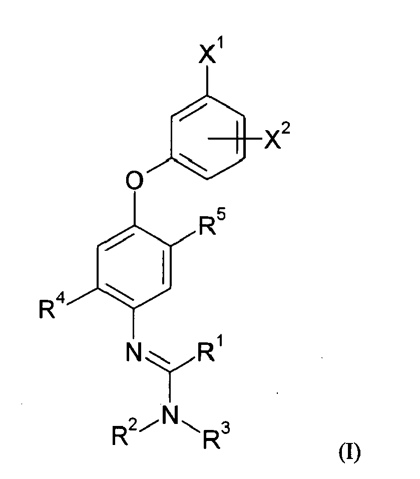 in welcher
R¹ ausgewählt ist aus Wasserstoff; linearen, verzweigten, C₁₋₁₂-Alkyl-, C₂₋₁₂-Alkenyl-, C₂₋₁₂-Alkinyl-Gruppen oder cyclischen C₃₋₁₂-Alkyl-, C₄₋₁₂-Alkenyl- , C₄₋₁₂-Alkinyl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und -CONR₂', substituiert sein können, wobei R' Wasserstoff oder eine C₁₋₁₂-Alkyl-Gruppe ist;
-SH; -SR", wobei R" eine lineare, verzweigte C₁₋₁₂-Alkyl-Gruppe, die mit einer oder mehreren Gruppen, die ausgewählt sind aus -R', -X, -OR', -SR', -NR'₂, -SiR'₃, -COOR', -CN und-CONR₂' substituiert sein kann, worin R' die obigen Bedeutungen hat;
R² ausgewählt ist aus linearen, verzweigten C₁₋₁₂-Alkyl-, C₂₋₁₂-Alkenyl-, C₂₋₁₂-Alkinyl-, cyclischen C₃₋₁₂-Alkyl-, C₄₋₁₂-Alkenyl-, C₄₋₁₂-Alkinyl-Gruppen oder C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und-CONR₂' substituiert sein können, worin R' die obigen Bedeutungen hat;
R³ ausgewählt ist aus -CN, -SH, -SR", -OR", -(C=O)-R", wobei R" die obigen Bedeutungen hat;
linearen, verzweigten C₂₋₁₂-Alkyl-, C₂₋₁₂-Alkenyl-, C₂₋₁₂-Alkinyl-, cyclischen C₃₋₁₂-Alkyl-, C₄₋₁₂-Alkenyl-, C₄₋₁₂-Alkinyl-Gruppen oder C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und-CONR₂' substituiert sein können, worin R' die obigen Bedeutungen hat;
oder in der
R² und R³,
R² und R¹ oder
R¹ und R³ mit den Atomen, an die sie gebunden sind, oder ggf. mit weiteren Atomen, ausgewählt aus N, O, P und S, einen vier- bis siebengliedrigen Ring bilden können, der wiederum mit R'-, OR'-, SR'-, NR'₂-, SiR'₃-Gruppen substituiert sein kann, worin R' die obigen Bedeutungen hat;
R⁴ und R⁵ unabhängig voneinander ausgewählt sind aus der Gruppe bestehend aus Wasserstoff, -X, -CN, -SH, -SR", -OR", -(C=O)-R", wobei R" die obigen Bedeutungen hat;
linearen, verzweigten C₁₋₁₂-Alkyl-, C₂₋₁₂-Alkenyl-, C₂₋₁₂-Alkinyl-, cyclischen C₃₋₁₂-Alkyl-, C₄₋₁₂-Alkenyl-, C₄₋₁₂-Alkinyl-Gruppen oder C₅₋₁₈-Aryl-, C₇₋₁₉-Aralkyl- oder C₇₋₁₉-Alkaryl-Gruppen, wobei im Ringsystem aller zuvor genannten cyclischen Gruppen ein oder mehrere C-Atome durch Heteroatome, ausgewählt aus N, O, P und S, ersetzt sein können und alle zuvor genannten Gruppen mit einer oder mehreren Gruppen, die ausgewählt sind aus -R', -X, -OR', -SR', -NR'₂, -SiR'₃, -COOR', -CN und-CONR₂' substituiert sein können, worin R' die obigen Bedeutungen hat;
X¹ ausgewählt ist aus der Gruppe bestehend aus F, Cl, Br und I;
X² sich entweder in 4- oder 5- Position der Phenyl-Gruppe befindet und unabhängig von X¹ ausgewählt ist aus der Gruppe bestehend aus F, Cl, Br und I;
wobei im Falle einer 3,5-Dihalosubstitution X¹ und X² nicht gleichzeitig Cl sind,
und deren Salze.

2. Dihalogenphenoxyphenylamidine gemäß Anspruch 1, wobei
R¹ ausgewählt aus der Gruppe bestehend aus Wasserstoff, einer Mercaptogruppe (-SH) oder C₁₋₈-Alkyl-Gruppen;
R² ausgewählt ist aus linearen oder verzweigten C₁₋₈-Alkyl-Gruppen;
R³ ausgewählt ist aus linearen, verzweigten und alicyclischen C₂₋₈-Alkyl-Gruppen;
oder wobei
R² und R³, gemeinsam mit dem N-Atom, an die sie gebunden sind oder mit weiteren Atomen, ausgewählt aus N, O, P und S, einen fünf- bis sechsgliedrigen Ring bilden können, der mit einer C₁₋₁₂-Alkyl-Gruppe substituiert sein kann; und
R⁴ und R⁵ unabhängig voneinander ausgewählt sind aus der Gruppe bestehend aus -X, linearen oder verzweigten, C₁₋₁₂-Alkyl-Gruppen und C₁₋₅-Haloalkyl-Gruppen;
X¹ ausgewählt ist aus der Gruppe bestehend aus F, Cl und Br;
X² sich entweder in 3-, 4- oder 5- Position der Phenyl-Gruppe befindet und unabhängig von X¹ ausgewählt ist aus der Gruppe bestehend aus F, Cl und Br;
wobei im Falle einer 3,5-Dihalosubstitution X¹ und X² nicht gleichzeitig Cl sind
und deren Salze.

3. Dihalogenphenoxyphenylamidine gemäß einem der Ansprüche 1 oder 2, wobei
R¹ ausgewählt ist aus der Gruppe bestehend aus Wasserstoff und Methyl;
R² ausgewählt ist aus der Gruppe bestehend aus Methyl und Ethyl;
R³ ausgewählt ist aus der Gruppe bestehend aus Ethyl und Cyclopropyl;
oder wobei
R² und R³ gemeinsam mit dem N-Atom, an das sie gebunden sind, einen Piperidyl- oder Pyrrolidyl-Rest bilden;
R⁴ und R⁵ unabhängig voneinander ausgewählt sind aus der Gruppe bestehend aus Cl- und F-Atomen und -CF₃, -CF₂H und Methyl-Gruppen;
X¹ ausgewählt ist aus der Gruppe bestehend aus F, Cl, Br und I;
X² sich entweder in 4- oder 5- Position der Phenyl-Gruppe befindet und ausgewählt ist aus der Gruppe bestehend aus F, Cl und Br;
wobei im Falle einer 3,5-Dihalosubstitution X¹ und X² nicht gleichzeitig Cl sind,
und deren Salzen.

4. Dihalogenphenoxyphenylamidine gemäß einem der Ansprüche 1 bis 3 ausgewählt aus der Gruppe bestehend aus N-Ethyl-N-methyl-N'-[4-(3,4-difluorphenoxy)-2,5-xylyl]formamidin; N-Methyl-N-propyl-N'-[4-(3,4-difluorphenoxy)-2,5-xylyl]formamidin;N-Cyclopropyl-N-methyl-N'-[4-(3,4-difluorphenoxy)-2,5-xylyl]formamidin; N,N-Diethyl-N'-[4-(3,4-difluorphenoxy)-2,5-xylyl]formamidin; N-Ethyl-N-propyl-N'-[4-(3,4-difluorphenoxy)-2,5-xylyl]formamidin; N-Pyrrolidinyl-N'-[4-(3,4-difluorphenoxy)-2,5-xylyl]formamidin; N-Piperidinyl-N'-[4-(3,4-difluorphenoxy)-2,5-xylyl]formamidin;N-(2,6-Dimethyl-morphlinyl)-N'-[4-(3,4-difluorphenoxy)-2,5-xylyl]formamidin; N-Ethyl-N-methyl-N'-[4-(3,5-difluorphenoxy)-2,5-xylyl]formamidin; N-Methyl-N-propyl-N'-[4-(3,5-difluorphenoxy)-2,5-xylyl]formamidin; N-Cyclopropyl-N-methyl-N'-[4-(3,5-difluorphenoxy)-2,5-xylyl]formamidin; N,N-Diethyl-N'-[4-(3,5-difluorphenoxy)-2,5-xylyl]formamidin; N-Ethyl-N-propyl-N'-[4-(3,5-difluorphenoxy)-2,5-xylyl]formamidin; N-Pyrrolidinyl-N'-[4-(3,5-difluorphenoxy)-2,5-xylyl]formamidin; N-Piperidinyl-N'-[4-(3,5-difluorphenoxy)-2,5-xylyl]formamidin; N-(2,6-Dimethylmorphlinyl)-N'-[4-(3,5-difluorphenoxy)-2,5-xylyl]formamidin; N-Ethyl-N-methyl-N'-[4-(3-chlor-4-fluorphenoxy)-2,5-xylyl]formamidin; N-Methyl-N-propyl-N'-[4-(3-chlor-4-fluorphenoxy)-2,5-xylyl]formamidin; N-Cyclopropyl-N-methyl-N'-[4-(3-chlor-4-fluorphenoxy)-2,5-xylyl]formamidin; N,N-Diethyl-N'-[4-(3-chlor-4-fluorphenoxy)-2,5-xylyl]formamidin; N-Ethyl-N-propyl-N'-[4-(3-chlor-4-fluorphenoxy)-2,5-xylyl]formamidin; N-Pyrrolidinyl-N'-[4-(3-chlor-4-fluorphenoxy)-2,5-xylyl]formamidin; N-Piperidinyl-N'-[4-(3-chlor-4-fluorphenoxy)-2,5-xylyl]formamidin; N-(2,6-Dimethyl-morpholinyl)-N'-[4-(3-chlor-4-fluorphenoxy)-2,5-xylyl]formamidin; N-Ethyl-N-methyl-N'-[4-(3-chlor-5-fluorphenoxy)-2,5-xylyl]formamidin; N-Methyl-N-propyl-N'-[4-(3-chlor-5-fluorphenoxy)-2,5-xylyl]formamidin; N-Cyclopropyl-N-methyl-N'-[4-(3-chlor-5-fluorphenoxy)-2,5-xylyl]formamidin; N,N-Diethyl-N'-[4-(3-chlor-5-fluorphenoxy)-2,5-xylyl]formamidin; N-Ethyl-N-propyl-N'-[4-(3-chlor-5-fluorphenoxy)-2,5-xylyl]formamidin; N-Pyrrolidinyl-N'-[4-(3-chlor-5-fluorphenoxy)-2,5-xylyl]formamidin; N-Piperidinyl-N'-[4-(3-chlor-5-fluorphenoxy)-2,5-xylyl]formamidin; N-(2,6-Dimethylmorphlinyl)-N'-[4-(3-chlor-5-fluorphenoxy)-2,5-xylyl]formamidin; N-Ethyl-N-methyl-N'-[4-(3-brom-4-fluorphenoxy)-2,5-xylyl]formamidin; N-Methyl-N-propyl-N'-[4-(3-brom-4-fluorphenoxy)-2,5-xylyl]formamidin; N-Cyclopropyl-N-methyl-N'-[4-(3-brom-4-fluorphenoxy)-2,5-xylyl]-formamidin; N,N-Diethyl-N'-[4-(3-brom-4-fluorphenoxy)-2,5-xylyl]formamidin; N-Ethyl-N-propyl-N'-[4-(3-brom-4-fluorphenoxy)-2,5-xylyl]formamidin; N-Pyrrolidinyl-N'-[4-(3-brom-4-fluorphenoxy)-2,5-xylyl]formamidin; N-Piperidinyl-N'-[4-(3-brom-4-fluorphenoxy)-2,5-xylyl]formamidin; N-(2,6-Dimethylmorpholinyl)-N'-[4-(3-brom-4-fluorphenoxy)-2,5-xylyl]formamidin; N-Ethyl-N-methyl-N'-[4-(3-brom-5-fluorphenoxy)-2,5-xylyl]formamidin; N-Methyl-N-propyl-N'-[4-(3-brom-5-fluorphenoxy)-2,5-xylyl]-formamidin; N-Cyclopropyl-N-methyl-N'-[4-(3-brom-5-fluorphenoxy)-2,5-xylyl]-formamidin; N,N-Diethyl-N'-[4-(3-brom-5-fluorphenoxy)-2,5-xylyl]formamidin; N-Ethyl-N-propyl-N'-[4-(3-brom-5-fluorphenoxy)-2,5-xylyl]formamidin; N-Pyrrolidinyl-N'-[4-(3-brom-5-fluorphenoxy)-2,5-xylyl]formamidin; N-Piperidinyl-N'-[4-(3-brom-5-fluorphenoxy)-2,5-xylyl]formamidin; N-(2,6-Dimethylmorphlinyl)-N'-[4-(3-brom-5-fluorphenoxy)-2,5-xylyl]formamidin; N-Ethyl-N-methyl-N'-[4-(3-iod-4-fluorphenoxy)-2,5-xylyl]formamidin; N-Methyl-N-propyl-N'-[4-(3-iod-4-fluorphenoxy)-2,5-xylyl]formamidin; N-Cyclopropyl-N-methyl-N'-[4-(3-iod-4-fluorphenoxy)-2,5-xylyl]formamidin; N,N-Diethyl-N'-[4-(3-iod-4-fluorphenoxy)-2,5-xylyl]formamidin; N-Ethyl-N-propyl-N'-[4-(3-iod-4-fluorphenoxy)-2,5-xylyl]formamidin; N-Pyrrolidinyl-N'-[4-(3-iod-4-fluorphenoxy)-2,5-xylyl]formamidin; N-Piperidinyl-N'-[4-(3-iod-4-fluorphenoxy)-2,5-xylyl]formamidin; N-(2,6-Dimethylmorphlinyl)-N'-[4-(3-iod-4-fluorphenoxy)-2,5-xylyl]formamidin; N-Ethyl-N-methyl-N'-[4-(3-iod-5-fluorphenoxy)-2,5-xylyl]formamidin; N-Methyl-N-propyl-N'-[4-(3-iod-5-fluorphenoxy)-2,5-xylyl]formamidin; N-Cyclopropyl-N-methyl-N'-[4-(3-iod-5-fluorphenoxy)-2,5-xylyl]formamidin; N,N-Diethyl-N'-[4-(3-iod-5-fluorphenoxy)-2,5-xylyl]formamidin; N-Ethyl-N-propyl-N'-[4-(3-iod-5-fluorphenoxy)-2,5-xylyl]formamidin; N-Pyrrolidinyl-N'-[4-(3-iod-5-fluorphenoxy)-2,5-xylyl]formamidin; N-Piperidinyl-N'-[4-(3-iod-5-fluorphenoxy)-2,5-xylyl]formamidin;N-(2,6-Dimethylmorphlinyl)-N'-[4-(3-iod-5-fluorphenoxy)-2,5-xylyl]formamidin; N-Ethyl-N-methyl-N'-[4-(3-fluor-4-chlorphenoxy)-2,5-xylyl]formamidin; N-Methyl-N-propyl-N'-[4-(3-fluor-4-chlorphenoxy)-2,5-xylyl]formamidin; N-Cyclopropyl-N-methyl-N'-[4-(3-fluor-4-chlorphenoxy)-2,5-xylyl]formamidin; N,N-Diethyl-N'-[4-(3-fluor-4-chlorphenoxy)-2,5-xylyl]formamidin; N-Ethyl-N-propyl-N'-[4-(3-fluor-4-chlorphenoxy)-2,5-xylyl]formamidin; N-Pyrrolidinyl-N'-[4-(3-fluor-4-chlorphenoxy)-2,5-xylyl]formamidin; N-Piperidinyl-N'-[4-(3-fluor-4-chlorphenoxy)-2,5-xylyl]formamidin; N-(2,6-Dimethylmorphlinyl)-N'-[4-(3-fluor-4-chlorphenoxy)-2,5-xylyl]formamidin; N-Ethyl-N-methyl-N'-[4-(3,4-dichlorphenoxy)-2,5-xylyl]formamidin; N-Methyl-N-propyl-N'-[4-(3,4-dichlorphenoxy)-2,5-xylyl]formamidin; N-Cyclopropyl-N-methyl-N'-[4-(3,4-dichlorphenoxy)-2,5-xylyl]formamidin; N,N-Diethyl-N'-[4-(3,4-dichlorphenoxy)-2,5-xylyl]formamidin; N-Ethyl-N-propyl-N'-[4-(3,4-dichlorphenoxy)-2,5-xylyl]-formamidin; N-Pyrrolidinyl-N'-[4-(3,4-dichlorphenoxy)-2,5-xylyl]formamidin; N-Piperidinyl-N'-[4-(3,4-dichlorphenoxy)-2,5-xylyl]formamidin; N-(2,6-Dimethylmorphlinyl)-N'-[4-(3,4-dichlorphenoxy)-2,5-xylyl]formamidin; N-Ethyl-N-methyl-N'-[4-(3-brom-4-chlorphenoxy)-2,5-xylyl]formamidin; N-Methyl-N-propyl-N'-[4-(3-brom-4-chlorphenoxy)-2,5-xylyl]formamidin; N-Cyclopropyl-N-methyl-N'-[4-(3-brom-4-chlorphenoxy)-2,5-xylyl]-formamidin; N,N-Diethyl-N'-[4-(3-brom-4-chlorphenoxy)-2,5-xylyl]formamidin; N-Ethyl-N-propyl-N'-[4-(3-brom-4-chlorphenoxy)-2,5-xylyl]formamidin; N-Pyrrolidinyl-N'-[4-(3-brom-4-chlorphenoxy)-2,5-xylyl]formamidin; N-Piperidinyl-N'-[4-(3-brom-4-chlorphenoxy)-2,5-xylyl]formamidin; N-(2,6-Dimethylmorphlinyl)-N'-[4-(3-brom-4-chlorphenoxy)-2,5-xylyl]formamidin; N-Ethyl-N-methyl-N'-[4-(3-brom-5-chlorphenoxy)-2,5-xylyl]formamidin; N-Methyl-N-propyl-N'-[4-(3-brom-5-chlorphenoxy)-2,5-xylyl]-formamidin; N-Cyclopropyl-N-methyl-N'-[4-(3-brom-5-chlorphenoxy)-2,5-xylyl]formamidin; N,N-Diethyl-N'-[4-(3-brom-5-chlorphenoxy)-2,5-xylyl]formamidin; N-Ethyl-N-propyl-N'-[4-(3-brom-5-chlorphenoxy)-2,5-xylyl]formamidin; N-Pyrrolidinyl-N'-[4-(3-brom-5-chlorphenoxy)-2,5-xylyl]formamidin; N-Piperidinyl-N'-[4-(3-brom-5-chlorphenoxy)-2,5-xylyl]formamidin; N-(2,6-Dimethylmorphlinyl)-N'-[4-(3-brom-5-chlorphenoxy)-2,5-xylyl]formamidin; N-Ethyl-N-methyl-N'-[4-(3-iod-4-chlorphenoxy)-2,5-xylyl]formamidin; N-Methyl-N-propyl-N'-[4-(3-iod-4-chlorphenoxy)-2,5-xylyl]formamidin; N-Cyclopropyl-N-methyl-N'-[4-(3-iod-4-chlorphenoxy)-2,5-xylyl]formamidin; N,N-Diethyl-N'-[4-(3-iod-4-chlorphenoxy)-2,5-xylyl]formamidin; N-Ethyl-N-propyl-N'-[4-(3-iod-4-chlorphenoxy)-2,5-xylyl]formamidin; N-Pyrrolidinyl-N'-[4-(3-iod-4-chlorphenoxy)-2,5-xylyl]formamidin; N-Piperidinyl-N'-[4-(3-iod-4-chlorphenoxy)-2,5-xylyl]formamidin; N-(2,6-Dimethylmorphlinyl)-N'-[4-(3-iod-4-chlorphenoxy)-2,5-xylyl]formamidin; N-Ethyl-N-methyl-N'-[4-(3-iod-5-chlorphenoxy)-2,5-xylyl]formamidin; N-Methyl-N-propyl-N'-[4-(3-iod-5-chlorphenoxy)-2,5-xylyl]formamidin; N-Cyclopropyl-N-methyl-N'-[4-(3-iod-5-chlorphenoxy)-2,5-xylyl]formamidin; N,N-Diethyl-N'-[4-(3-iod-5-chlorphenoxy)-2,5-xylyl]formamidin; N-Ethyl-N-propyl-N'-[4-(3-iod-5-chlorphenoxy)-2,5-xylyl]formamidin; N-Pyrrolidinyl-N'-[4-(3-iod-5-chlorphenoxy)-2,5-xylyl]formamidin; N-Piperidinyl-N'-[4-(3-iod-5-chlorphenoxy)-2,5-xylyl]formamidin; N-(2,6-Dimethylinorphlinyl)-N'-[4-(3-iod-5-chlorphenoxy)-2,5-xylyl]formamidin; N-Ethyl-N-methyl-N'-[4-(3-fluor-4-bromphenoxy)-2,5-xylyl]formamidin; N-Methyl-N-propyl-N'-[4-(3-fluor-4-bromphenoxy)-2,5-xylyl]formamidin; N-Cyclopropyl-N-methyl-N'-[4-(3-fluor-4-bromphenoxy)-2,5-xylyl]formamidin; N,N-Diethyl-N'-[4-(3-fluor-4-bromphenoxy)-2,5-xylyl]formamidin; N-Ethyl-N-propyl-N'-[4-(3-fluor-4-bromphenoxy)-2,5-xylyl]formamidin; N-Pyrrolidinyl-N'-[4-(3-fluor-4-bromphenoxy)-2,5-xylyl]formamidin; N-Piperidinyl-N'-[4-(3-fluor-4-bromphenoxy)-2,5-xylyl]formamidin; N-(2,6-Dimethylmorphlinyl)-N'-[4-(3-fluor-4-bromphenoxy)-2,5-xylyl]formamidin; N-Ethyl-N-methyl-N'-[4-(3-chloro-4-bromphenoxy)-2,5-xylyl]formamidin; N-Methyl-N-propyl-N'-[4-(3-chloro-4-bromphenoxy)-2,5-xylyl]formamidin; N-Cyclopropyl-N-methyl-N'-[4-(3-chloro-4-bromphenoxy)-2,5-xylyl]formamidin; N,N-Diethyl-N'-[4-(3-chloro-4-bromphenoxy)-2,5-xylyl]formamidin; N-Ethyl-N-propyl-N'-[4-(3-chloro-4-bromphenoxy)-2,5-xylyl]formamidin; N-Pyrrolidinyl-N'-[4-(3-chloro-4-bromphenoxy)-2,5-xylyl]formamidin; N-Piperidinyl-N'-[4-(3-chloro-4-bromphenoxy)-2,5-xylyl]formamidin; N-(2,6-Dimethylmorphlinyl)-N'-[4-(3-chloro-4-bromphenoxy)-2,5-xylyl]-formamidin;N-Ethyl-N-methyl-N'-[4-(3,4-dibromphenoxy)-2,5-xylyl]formamidin; N-Methyl-N-propyl-N'-[4-(3,4-dibromphenoxy)-2,5-xylyl]formamidin; N-Cyclopropyl-N-methyl-N'-[4-(3,4-dibromphenoxy)-2,5-xylyl]formamidin; N,N-Diethyl-N'-[4-(3,4-dibromphenoxy)-2,5-xylyl]formamidin; N-Ethyl-N-propyl-N'-[4-(3,4-dibromphenoxy)-2,5-xylyl]formamidin; N-Pyrrolidinyl-N'-[4-(3,4-dibromphenoxy)-2,5-xylyl]formamidin; N-Piperidinyl-N'-[4-(3,4-dibromphenoxy)-2,5-xylyl]formamidin; N-(2,6-Dimethylmorphlinyl)-N'-[4-(3,4-dibromphenoxy)-2,5-xylyl]formamidin; N-Ethyl-N-methyl-N'-[4-(3,5-dibromphenoxy)-2,5-xylyl]formamidin; N-Methyl-N-propyl-N'-[4-(3,5-dibromphenoxy)-2,5-xylyl]formamidin; N-Cyclopropyl-N-methyl-N'-[4-(3,5-dibromphenoxy)-2,5-xylyl] formamidin; N,N-Diethyl-N'-[4-(3,5-dibromphenoxy)-2,5-xylyl]formamidin; N-Ethyl-N-propyl-N'-[4-(3,5-dibromphenoxy)-2,5-xylyl]formamidin; N-Pyrrolidinyl-N'-[4-(3,5-dibromphenoxy)-2,5-xylyl]formamidin; N-Piperidinyl-N'-[4-(3,5-dibromphenoxy)-2,5-xylyl]formamidin; N-(2,6-Dimethylmorphlinyl)-N'-[4-(3,5-dibromphenoxy)-2,5-xylyl]formamidin; N-Ethyl-N-methyl-N'-[4-(3-iod-4-bromphenoxy)-2,5-xylyl]formamidin; N-Methyl-N-propyl-N'-[4-(3-iod-4-bromphenoxy)-2,5-xylyl]formamidin; N-Cyclopropyl-N-methyl-N'-[4-(3-iod-4-bromphenoxy)-2,5-xylyl]formamidin; N,N-Diethyl-N'-[4-(3-iod-4-bromphenoxy)-2,5-xylyl]formamidin; N-Ethyl-N-propyl-N'-[4-(3-iod-4-bromphenoxy)-2,5-xylyl]formamidin; N-Pyrrolidinyl-N'-[4-(3-iod-4-bromphenoxy)-2,5-xylyl]formamidin; N-Piperidinyl-N'-[4-(3-iod-4-bromphenoxy)-2,5-xylyl]formamidin; N-(2,6-Dimethylmorphlinyl)-N'-[4-(3-iod-4-bromphenoxy)-2,5-xylyl]formamidin; N-Ethyl-N-methyl-N'-[4-(3-iod-5-bromphenoxy)-2,5-xylyl]formamidin; N-Methyl-N-propyl-N'-[4-(3-iod-5-bromphenoxy)-2,5-xylyl]formamidin; N-Cyclopropyl-N-methyl-N'-[4-(3-iod-5-bromphenoxy)-2,5-xylyl]formamidin; N,N-Diethyl-N'-[4-(3-iod-5-bromphenoxy)-2,5-xylyl]formamidin; N-Ethyl-N-propyl-N'-[4-(3-iod-5-bromphenoxy)-2,5-xylyl]formamidin; N-Pyrrolidinyl-N'-[4-(3-iod-5-bromphenoxy)-2,5-xylyl]formamidin; N-Piperidinyl-N'-[4-(3-iod-5-bromphenoxy)-2,5-xylyl]formamidin; N-(2,6-Dimethylmorphlinyl)-N'-[4-(3-iod-5-bromphenoxy)-2,5-xylyl]formamidin; N-Ethyl-N-methyl-N'-[4-(3-fluor-4-iodphenoxy)-2,5-xylyl]formamidin; N-Methyl-N-propyl-N'-[4-(3-fluor-4-iodphenoxy)-2,5-xylyl]formamidin;N-Cyclopropyl-N-methyl-N'-[4-(3-fluor-4-iodphenoxy)-2,5-xylyl]formamidin; N,N-Diethyl-N'-[4-(3-fluor-4-iodphenoxy)-2,5-xylyl]formamidin; N-Ethyl-N-propyl-N'-[4-(3-fluor-4-iodphenoxy)-2,5-xylyl]formamidin; N-Pyrrolidinyl-N'-[4-(3-fluor-4-iodphenoxy)-2,5-xylyl]formamidin; N-Piperidinyl-N'-[4-(3-fluor-4-iodphenoxy)-2,5-xylyl]formamidin; N-(2,6-Dimethylmorphlinyl)-N'-[4-(3-fluor-4-iodphenoxy)-2,5-xylyl]formamidin; N-Ethyl-N-methyl-N'-[4-(3-chloro-4-iodphenoxy)-2,5-xylyl]formamidin; N-Methyl-N-propyl-N'-[4-(3-chloro-4-iodphenoxy)-2,5-xylyl]formamidin; N-Cyclopropyl-N-methyl-N'-[4-(3-chloro-4-iodphenoxy)-2,5-xylyl]formamidin; N,N-Diethyl-N'-[4-(3-chloro-4-iodphenoxy)-2,5-xylyl]formamidin; N-Ethyl-N-propyl-N'-[4-(3-chloro-4-iodphenoxy)-2,5-xylyl]formamidin; N-Pyrrolidinyl-N'-[4-(3-chloro-4-iodphenoxy)-2,5-xylyl]formamidin; N-Piperidinyl-N'-[4-(3-chloro-4-iodphenoxy)-2,5-xylyl]formamidin; N-(2,6-Dimethylmorphlinyl)-N'-[4-(3-chloro-4-iodphenoxy)-2,5-xylyl]formamidin; N-Ethyl-N-methyl-N'-[4-(3-brom-4-iodphenoxy)-2,5-xylyl]formamidin; N-Methyl-N-propyl-N'-[4-(3-brom-4-iodphenoxy)-2,5-xylyl]formamidin, N-Cyclopropyl-N-methyl-N'-[4-(3-brom-4-iodphenoxy)-2,5-xylyl]-formamidin; N,N-Diethyl-N'-[4-(3-brom-4-iodphenoxy)-2,5-xylyl]formamidin; N-Ethyl-N-propyl-N'-[4-(3-brom-4-iodphenoxy)-2,5-xylyl]formamidin; N-Pyrrolidinyl-N'-[4-(3-brom-4-iodphenoxy)-2,5-xylyl]formamidin; N-Piperidinyl-N'-[4-(3-brom-4-iodphenoxy)-2,5-xylyl]formamidin; N-(2,6-Dimethylmorphlinyl)-N'-[4-(3-brom-4-iodphenoxy)-2,5-xylyl]formamidin; N-Ethyl-N-methyl-N'-[4-(3,4-diiodphenoxy)-2,5-xylyl]formamidin; N-Methyl-N -propyl-N' -[4-(3, 4-diiodphenoxy)-2,5 -xylyl]formamidin; N-Cyclopropyl-N-methyl-N'-[4-(3,4-diiodphenoxy)-2,5-xylyl]formamidin; N,N-Diethyl-N'-[4-(3,4-diiodphenoxy)-2,5-xylyl]formamidin; N-Ethyl-N-propyl-N'-[4-(3,4-diiodphenoxy)-2,5-xylyl]-formamidin; N-Pyrrolidinyl-N'-[4-(3,4-diiodphenoxy)-2,5-xylyl]formamidin; N-Piperidinyl-N'-[4-(3,4-diiodphenoxy)-2,5-xylyl]formamidin; N-(2,6-Dimethylmorphlinyl)-N'-[4-(3,4-diiodphenoxy)-2,5-xylyl]formamidin; N-Ethyl-N-methyl-N'-[4-(3,5-diiodphenoxy)-2,5-xylyl]formamidin; N-Methyl-N-propyl-N'-[4-(3,5-diiodphenoxy)-2,5-xylyl]formamidin;N-Cyclopropyl-N-methyl-N'-[4-(3,5-diiodphenoxy)-2,5-xylyl]formamidin; N,N-Diethyl-N'-[4-(3,5-diiodphenoxy)-2,5-xylyl]formamidin; N-Ethyl-N-propyl-N'-[4-(3,5-diiodphenoxy)-2,5-xylyl]formamidin; N-Pyrrolidinyl-N'-[4-(3,5-diiodphenoxy)-2,5-xylyl]formamidin; N-Piperidinyl-N'-[4-(3,5-diiodphenoxy)-2,5 xylyl]formamidin; N-(2,6-Dimethylmorphlinyl)-N'-[4-(3,5-diiodphenoxy)-2,5-xylyl]formamidin.

5. Verfahren zum Herstellen der Phenoxyamidine gemäß einem der Ansprüch 1 bis 4 umfassend wenigstens einen der folgenden Schritte (a) bis (j):
(a) Umsetzung von Nitrobenzolderivaten der Formel (III) mit Dihalogenphenolen der Formel (II) gemäß dem nachfolgenden Reaktionsschema: 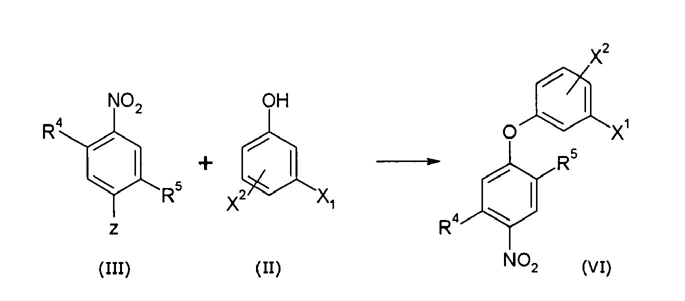
(b) Umsetzung von Nitrophenolderivaten der Formel (V) mit Dihalogenphenylderivaten der Formel (IV) gemäß dem nachfolgenden Reaktionsschema: 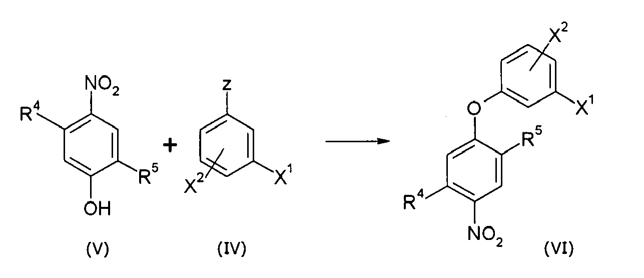
(c) Umsetzung von Anilinen der Formel (VII) mit Dihalogenphenolen der Formel (II) gemäß dem nachfolgenden Reaktionsschema: 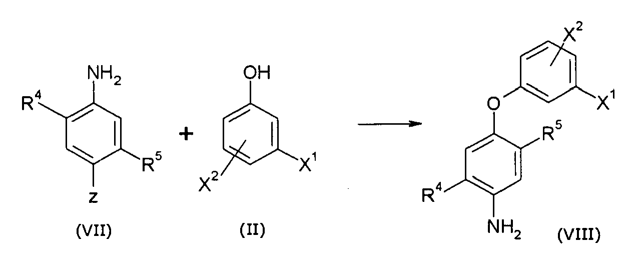
(d) Umsetzung von Aminophenolen der Formel (XII) mit Dihalogenphenylderivaten der Formel (IV) gemäß dem nachfolgenden Reaktionsschema: 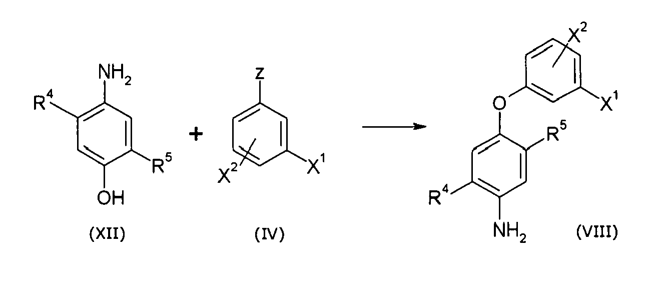
(e) Reduktion der Nitrophenylether der Formel (VI) zu Anilinethern der Formel (VIII) gemäß dem nachfolgenden Reaktionsschema: 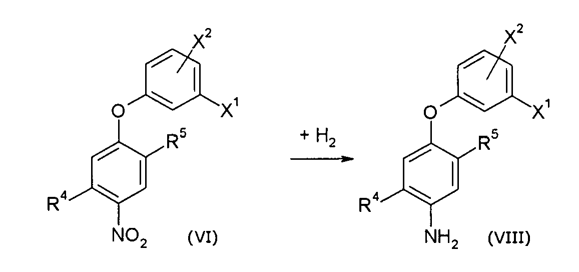
(f) Umsetzung der Anilinether der Formel (VIII) mit
(i) Aminoacetalen der Formel (XIII) oder
(ii) mit Amiden der Formel (XIV) oder
(iii) mit Aminen der Formel (XV) in Gegenwart von Orthoestern der Formel (XVI)
gemäß dem nachfolgenden Reaktionsschema: 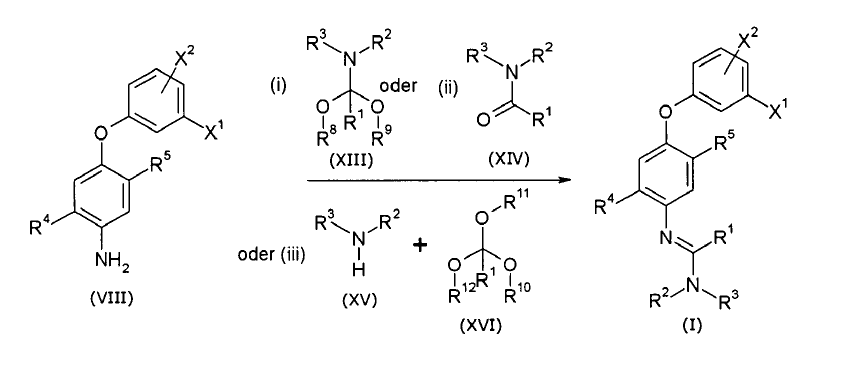
(g) Umsetzung der Aminophenole der Formel (XII) mit
(i) Aminoacetalen der Formel (XIII) oder
(ii) mit Amiden der Formel (XIV) oder
(iii) mit Aminen der Formel (XV) in Gegenwart von Orthoestern der Formel (XVI)
gemäß dem nachfolgenden Reaktionsschema: 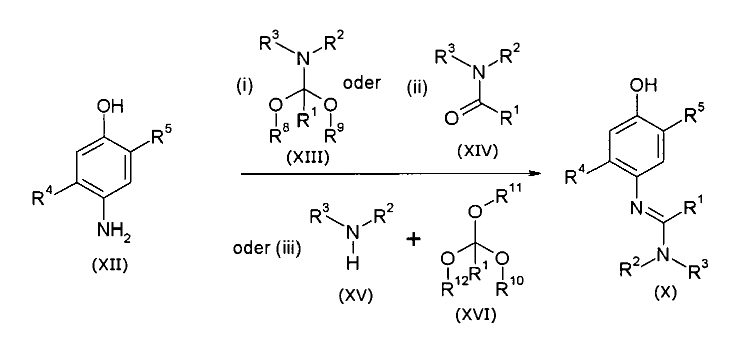
(h) Umsetzung der Aminophenole der Formel (VII) mit
(i) Aminoacetalen der Formel (XIII) oder
(ii) mit Amiden der Formel (XIV) oder
(iii) mit Aminen der Formel (XV) in Gegenwart von Orthoestern der Formel (XVI)
gemäß dem nachfolgenden Reaktionsschema: 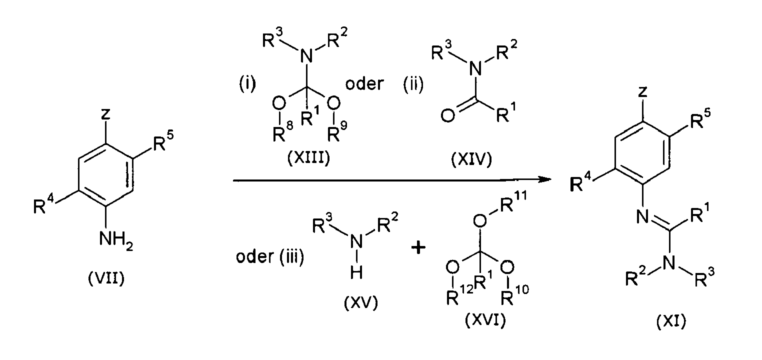
(i) Umsetzung von Amidinen der Formel (XI) mit dihalogensubstituierten Phenolen der Formel (II) gemäß dem nachfolgenden Reaktionsschema: 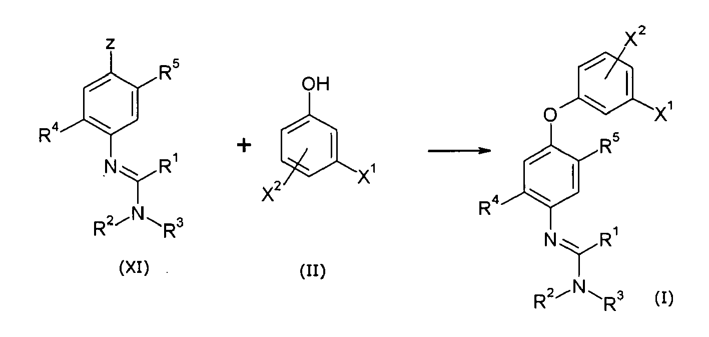
(j) Umsetzung von Amidinen der Formel (XI) mit dihalogensubstituierten Phenylderivaten der Formel (IV) gemäß dem nachfolgenden Reaktionsschema: 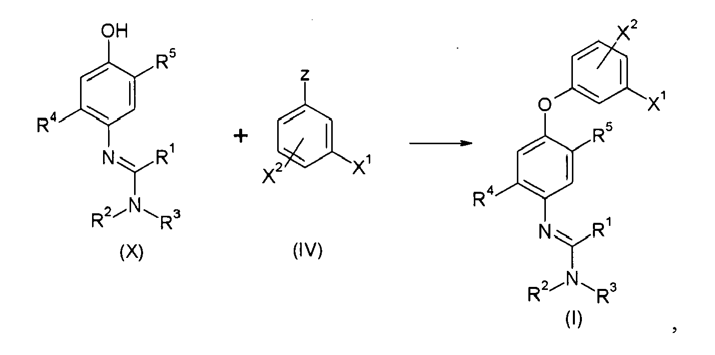
wobei in den obigen Schemata
Z eine Austrittsgruppe ist;
X¹, X² und R¹ bis R⁵ die obigen Bedeutungen haben;
R⁶ bis R⁹ unabhängig voneinander ausgewählt sind aus der Gruppe bestehend aus Wasserstoff, C₁₋₁₂-Alkyl-, C₂₋₁₂-Alkenyl- C₂₋₁₂-Alkinyl- oder C₅₋₁₈-Aryl- oder C₇₋₁₉-Arylalkyl-Gruppen und gemeinsam mit den O-Atomen, an die sie gebunden sind einen fünf-, sechs- oder siebengliedrigen carbo- oder hetero-cyclischen Ring bilden können;
R¹⁰, R¹¹ und R¹² unabhängig voneinander ausgewählt sind aus der Gruppe bestehend aus Wasserstoff, C₁₋₁₂-Alkyl-, C₂₋₁₂-Alkenyl-, C₂₋₁₂-Alkinyl- oder C₅₋₁₈-Aryl- oder C₇₋₁₉-Arylalkyl-, C₇₋₁₉-Alkylaryl-Gruppen und jeweils R¹⁰ mit R¹², R¹⁰ mit R¹¹ oder R¹¹ mit R¹² gemeinsam mit den O-Atomen, an die sie gebunden sind einen fünf-, sechs- oder siebengliedrigen Carbo- oder heterocyclischen Ring bilden können.

6. Nitrophenylether der Formel (VI); 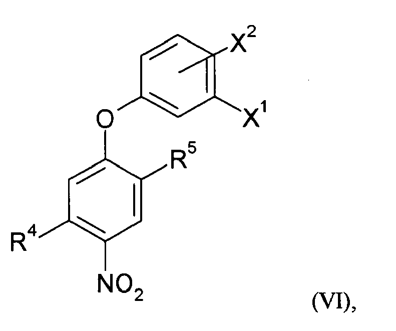 in der
R⁴,R⁵ X¹ und X² die obigen Bedeutungen haben;
und wobei im Falle einer 3,5-Dihalosubstitution X¹ und X² nicht gleichzeitig Cl sind.

7. Verwendung der Nitrophenylether der Formel (VI), gemäß Anspruch 6 zur Herstellung der Dihalogenphenoxyphenylamidine gemäß Formel (I).

8. Anilinether der Formel (VIII) 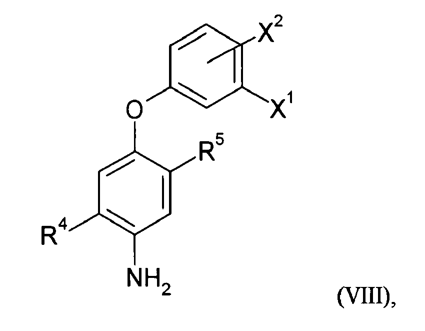 in der
R⁴, R⁵ X¹ und X² die obigen Bedeutungen haben;
und wobei im Falle einer 3,5-Dihalosubstitution X¹ und X² nicht gleichzeitig Cl sind.

9. Verwendung der Nitrophenylether der Formel (VIII), gemäß Anspruch 7 zur Herstellung der Dihalogenphenoxyphenylamidine gemäß Formel (I).

10. Aminoacetal der Formel (XIII) 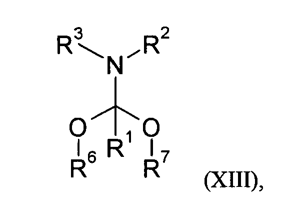 in der
R¹ bis R⁷ die obigen Bedeutungen ben;

11. Verwendung der Aminoacetale der Formel (XIII), gemäß Anspruch 10 zur Herstellung der Dihalogenphenoxyphenylamidine gemäß Formel (I).

12. Mittel zum Bekämpfen unerwünschter Mikroorganismen, umfassend wenigstens ein Dihalogenphenoxyphenylamidin gemäß einem der Ansprüche 1 bis 4.

13. Verwendung eines Amidins gemäß irgendeinem der Ansprüche 1 bis 4 oder Mischungen dieser zum Bekämpfen unerwünschter Mikroorganismen.

14. Verfahren zum Bekämpfen unerwünschter Mikroorganismen, **dadurch gekennzeichnet, dass** wenigstens ein Dihalogenphenoxyphenylamidin gemäß einem der Ansprüche 1 bis 4 auf die Mikroorganismen und/oder in deren Lebensraum ausgebracht wird.

15. Saatgut, welches mit wenigstens einem Amidin gemäß einem der Ansprüche 1 bis 4 behandelt ist.

16. Verwendung von Amidinen gemäß einem der Ansprüche 1 bis 4 zur Behandlung von Saatgut.

17. Verwendung von Amidinen gemäß einem der Ansprüche 1 bis 4 zur Behandlung von transgenen Pflanzen.

18. Verwendung von Amidinen gemäß einem der Ansprüche 1 bis 4 zur Behandlung von Saatgut transgener Pflanzen.

19. Verfahren zum Schutz von Saatgut vor unerwünschten Mikroorganismen durch Verwendung eines mit wenigstens einem Amidin gemäß einem der Ansprüche 1 bis 4 behandelten Saatguts.
